# EUROPEAN PATENT SPECIFICATION

(11) **EP 3 369 740 B1**
(45) Date of publication and mention of the grant of the patent: **25.11.2020**
(21) Application number: 18165697.6
(22) Date of filing: 10.06.2015
(51) Int. Cl.: C07H 19/06, C07H 1/00, A61K 31/7068, A61P 35/00

(54) **NEW CYTIDINE DERIVATIVE DIMERS AND APPLICATIONS THEREOF**
NEUE CYTIDINDERIVATDIMERE UND ANWENDUNGEN DAVON
NOUVEAUX DIMÈRES DÉRIVÉS DE CYTIDINE ET LEURS APPLICATIONS

(30) Priority: 17.11.2014 CN 201410652724; 09.04.2015 CN 201510167580
(43) Date of publication of application: 05.09.2018
(62) Divisional of application: 15860643.4
(73) Proprietor: Changzhou Fangyuan Pharmaceutical Co., Ltd., Jiangsu 213125 (CN); INNER MONGOLIA PUYIN PHARMACEUTICAL CO., LTD., Chifeng, Inner Mongolia 024000 (CN)
(72) Inventor: YANG, Daria, Changzhou, Jiangsu 213022 (CN); WANG, Haidong, Changzhou, Jiangsu 213022 (CN); WANG, Huijuan, Changzhou, Jiangsu 213022 (CN)
(74) Representative: Sun, Yiming

(56) References cited:
- WO-A1-2014/078295
- WO-A2-2004/041203
- KONDO K ET AL: "STUDIES ON BIOLOGICALLY ACTIVE NUCLEOSIDES AND NUCLEOTIDES. Ö5. SYNTHESIS AND ANTITUMOR ACTIVITY OF SOME 2,2'-ANHYDRO-1-(3',5'-DI-O-ACYL-BETA-D-ARA BINOFURANOSYL)CYTOSINE SALTS AND 2,2'-ANHYDRO-1-(3'-O-ACYL-BETA-D-ARABINOFU RANOSYL)CYTOSINE 5'PHOSPHATES", JOURNAL OF MEDICINAL CHEMISTRY, AMERICAN CHEMICAL SOCIETY, vol. 22, no. 6, 1 June 1979 (1979-06-01), pages 639-646, XP000573809, ISSN: 0022-2623, DOI: 10.1021/JM00192A007
- THEODOROS KARAMPELAS ET AL: "GnRH-Gemcitabine Conjugates for the Treatment of Androgen-Independent Prostate Cancer: Pharmacokinetic Enhancements Combined with Targeted Drug Delivery", BIOCONJUGATE CHEMISTRY, vol. 25, no. 4, 1 January 2014 (2014-01-01), pages 813-823, XP055396050, US ISSN: 1043-1802, DOI: 10.1021/bc500081g

## Description

### TECHNICAL FIELD

The present invention relates to an antitumor compound and, more particularly, relates to a new cytidine derivative dimer and applications thereof.

### BACKGROUND TECHNOLOGIES

Cancer is one of the common diseases that threaten human health. The mortality rate of cancer ranks the highest among other diseases. Currently clinical antitumor drugs face prominent toxicity issues in chemotherapy. Nowadays, an important research topic on antitumor drugs is to improve the therapeutic effect while reducing the toxicity of the drugs at the same time.

Thus, WO 2014/078295 A1 discloses compounds according to formula I 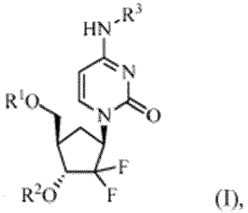 and pharmaceutically acceptable salts thereof. For compounds of formula I, R1 and R2 are independently selected from the group consisting of H, -C(=O)(CH2)2-aryl, and C(=O)(CH2)nC(=O)NH -aryl. The subscript n is from 2 to 6. R3 is selected from the group consisting of H and -C(=O)-O-R4; and R4 is selected from the group consisting of alkyl, substituted alkyl, alkenyl, substituted alkenyl, alkynyl, substituted alkynyl, arylalkyl, substituted arylalkyl, cycloheteroalkyl, substituted cycloheteroalkyl, heteroalkyl and substituted heteroalkyl. At least one of R1 and R2 is other than H. Pharmaceutical compositions, methods for inhibiting the growth of cancer cells, and methods for the treatment of cancer are also disclosed.

WO 2004/041203 A1 discloses gemcitabine prodrugs, methods of making gemcitabine prodrugs, pharmaceutical compositions of gemcitabine prodrugs and methods of using gemcitabine prodrugs and pharmaceutical compositions thereof to treat or prevent diseases or disorders such as cancer or viral infections.

Existing cytidine compounds are mainly used for treating blood tumors. Certain cytidine compounds are also used for solid tumors. However, problems arise due to high toxicity, narrow scope of application and poor therapeutic effect.

Further, human body is prone to producing drug resistance to existing cytidine compounds, resulting in failure of treatment and tumor recurrence.

### SUMMARY OF THE INVENTION

The technical problem to be solved by the present invention is to provide a new cytidine derivative dimer and applications thereof with high efficacy, high anti-tumor activity and low toxicity.

The technical solution to achieve the object of the present invention includes a new cytidine derivative dimer having the following formula (I): 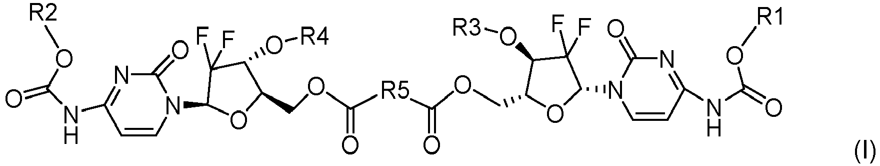 where R1 is C₁-C₁₀ alkyl, C₁-C₁₀ substituted alkyl, -(CH₂)ₙ-Ph, or substituted -(CH₂)ₙ-Ph. In the -(CH₂)ₙ-Ph, n=0, 1, 2, 3∼10, and Ph is phenyl. A carbon chain of the substituted alkyl is independently substituted by one, two, or three of halogen, cyano group, nitro group, amino group, hydroxyl group, or carboxyl group. In the substituted -(CH₂)ₙ-Ph, where n=0, 1, 2, 3∼10, and a carbon chain or a phenyl ring of which is independently substituted by one, two, or three of halogen, cyano group, nitro group, amino group, hydroxyl group, or carboxyl group. R1 is, preferably, C₁-C₁₀ alkyl or -(CH₂)ₙ-Ph, where n=0, 1, 2, 3∼10; more preferably, C₁-C₄ alkyl or - (CH₂)ₙ-Ph, where n=0, 1, 2, 3; and furthermore preferably, n-butyl or benzyl.

R2 is C₁-C₁₀ alkyl, C₁-C₁₀ substituted alkyl, -(CH₂)ₙ-Ph, or substituted -(CH₂)ₙ-Ph. In the -(CH₂)ₙ-Ph, n=0, 1, 2, 3∼10. A carbon chain of the substituted alkyl is independently substituted by one, two, or three of halogen, cyano group, nitro group, amino group, hydroxyl group, or carboxyl group. In the substituted -(CH₂)ₙ-Ph, where n=0, 1, 2, 3∼10, and a carbon chain or a phenyl ring of which is independently substituted by one, two, or three of halogen, cyano group, nitro group, amino group, hydroxyl group, or carboxyl group. R2 is, preferably, C₁-C₁₀ alkyl or -(CH₂)ₙ-Ph, where n=0, 1, 2, 3∼10; more preferably, C₁-C₄ alkyl or -(CH₂)ₙ-Ph, where n=0, 1, 2, 3; and furthermore preferably, n-butyl or benzyl.

As a more preferable choice, R1 and R2 are the same.

R3 is hydrogen, alkoxycarbonyl or substituted alkoxycarbonyl, where a substituent of the substituted alkoxycarbonyl is halogen, cyano group, nitro group, amino group, hydroxyl group, or carboxyl group. R3 is, preferably, H or alkoxycarbonyl; and more preferably, H or n-butoxycarbonyl.

R4 is hydrogen, alkoxycarbonyl or substituted alkoxycarbonyl, where a substituent of the substituted alkoxycarbonyl is halogen, cyano group, nitro group, amino group, hydroxyl group, or carboxyl group. R4 is, preferably, H or alkoxycarbonyl; and more preferably, H or n-butoxycarbonyl.

As a more preferable choice, R3 and R4 are both hydrogen.

R5 is -(CH₂)ₙ-, where n is from 1 to 15; or substituted -(CH₂)ₙ- with a substituent on a carbon chain thereof, the substituent of which is phenyl group, substituted phenyl group, cyano group, nitro group, amino group, hydroxyl group, carboxyl group, or - (CH₂)ₙ-X₁-X₂-. In the -(CH₂)ₙ-X₁-X₂-, n=0, 1, 2, or 3, X₁ is O or S, and X₂ is -(CH₂)ₙ-Ph, where n=0, 1, 2, or 3, or X₂ is pyrimidyl, pyranyl, imidazolyl, pyrazinyl, or pyridyl. R5 is, preferably, -(CH₂)ₙ-, n being from 1 to 15 or -(CH₂)ₙ-X₁-X₂-, where n=0, 1, 2, or 3, X₁ is O or S, and X₂ is Ph; more preferably, -(CH₂)ₙ-, n being from 1 to 5 or -(CH₂)-O-Ph-; and furthermore preferably, -(CH₂)₂- or -(CH₂)₃-.

Applications of the aforementioned new cytidine derivative dimer or a salt form thereof in preparing drugs for inhibiting tumor.

The tumor is blood tumor or malignant solid tumor.

A pharmaceutical composition includes: a cytidine derivative dimer as shown by formula (I) or a pharmaceutically acceptable salt thereof as an active ingredient; and one or more of medicinal carriers or excipients.

The form of the composition is an injection form or an oral dosage form. The oral dosage form includes: tablet, powder, granule, capsule, pellets formulation, solution, suspension, emulsion, syrups or elixirs; and the injection form includes solution-type injection, a suspension-type injection, an emulsion-type injection, or injectable sterile powder.

A method for preparing the aforementioned new cytidine derivative dimer, including the following steps.
(1) A compound having a general formula (II) is prepared. 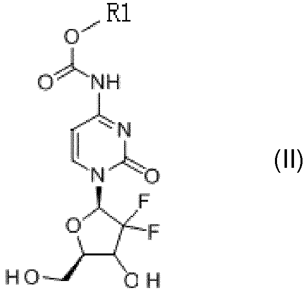
(2) A compound having a general formula (III) is prepared. 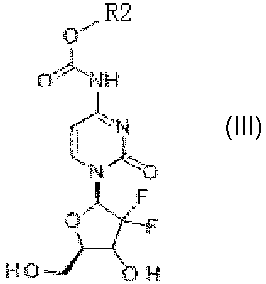
(3) The compound having the general formula (II) is mixed with sodium carbonate to add to a system including 1,4-dioxane and water. (Boc)₂O is further added for a reaction. According to TLC (thin-layer chromatography) measurement, when the reaction is measured to be completed, a reaction product is extracted, washed, and dried, and then concentrated to dryness under a reduced pressure. Trichloromethane (i.e., chloroform) is added with the dried compound, and added with pyridine and dianhydrides R₅(CO)₂O for an overnight reaction; a reaction product is concentrated to obtain a viscous oil. The viscous oil is purified by column chromatography to obtain a compound having a general formula (IV). 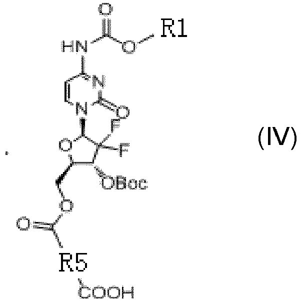
(4) After mixing the obtained compound having a general formula (IV), the obtained compound having a general formula (III), and DCC, the mixture is added to dichloromethane, and DMAP is added. According to TLC measurement, when the reaction is measured to be completed, a reaction product is washed, dried, and concentrated to form a dried compound. TFA and DCM are then added, and stirred at room temperature. After cooling with ice bath, white solid is filtered out. The filtrate is concentrated to obtain a viscous oil, which is purified by column chromatography to obtain the product.

The present invention also relates to the treatment of cancer. In particular, the present invention is targeted to treat a subject suffering from tumor, including a mammal, especially a human. Administering a therapeutically effective dose of the new compound (I) to the subject over a period of time produces an anti-tumor effect.

By its broadest definition, cancer generally refers to malignant neoplasm, which is an abnormal tissue that grows at a rate that is different from normal tissue and continues to overgrow in the same way after the induction of the stimulus ceases. In addition, this abnormal tissue has no purpose, absorbs nutrients from the host, and is almost autonomous. Cancer can also refer to malignant tumor. Further discussion of neoplasm can be found in Chapter 8 of book Robbins Pathologic Basis of Disease, sixth edition, by RS Cotran, V. Kumar, and T. Collins RS Cotran (published by WB Saunders Company). Examples of cancer types that can be treated by administration of the disclosed compound, such as a malignant tumor or neoplasm.

The disclosed new compounds are effective in the treatment of neoplasms, including leukemia and solid tumors. Solid tumors include tumors in colon, colon, rectum, ovary, breast, prostate, lung, kidney and melanoma. The range of drug dosage depends on route of administration and age, body weight and condition of the patient. The route of administration for the compound may be a parenteral route, including intramuscular injection, intravenous injection, or venous injection.

As used herein, a patient or a subject is a vertebrate with a cancer or other disease. Preferably, the subject is a warm-blooded animal, a mammal in particular, including both human and non-human mammals. Examples of non-human mammals include, farm animals such as cattle, sheep, pigs, goats, horses, and llamas, and pets such as dogs and cats. More preferably, the subject is a human. The present invention achieves an anti-tumor effect by administering to a subject a therapeutically effective dose of the compound over a period of time.

For mammals, including humans, an effective dose can be determined based on body surface area. In Cancer Chemother. Rep., 50 (4): 219 (1966), E. J. Freireich et al. illustrate relationships that the dose varies according to the size and type of animals and humans (on the basis of body surface area mg/m²). The body surface area can be approximately determined according to the height and weight of the individual (see, for example, Scientific Tables, Geigy Pharmaceuticals, Ardsley, N.Y. pp. 537-538 (1970)). A suitable dosage range may be from 1 to 1000 mg of the disclosed compound per square meter of body surface area. That is, the dose is 50-500 mg/m².

The present invention has several advantageous effects: (1) by molecular optimization designing of cytidine-based compounds, the prepared new cytidine derivative dimers of the present invention show significant inhibiting effects on HCT-116 human colon cancer cells, and exhibit strong growth inhibiting effects on HCT-116 human colon cancer xenografts grown in nude mice; the disclosed new cytidine derivative dimers provide high anti-tumor activity with low toxicity.

### BRIEF DESCRIPTION OF THE DRAWINGS

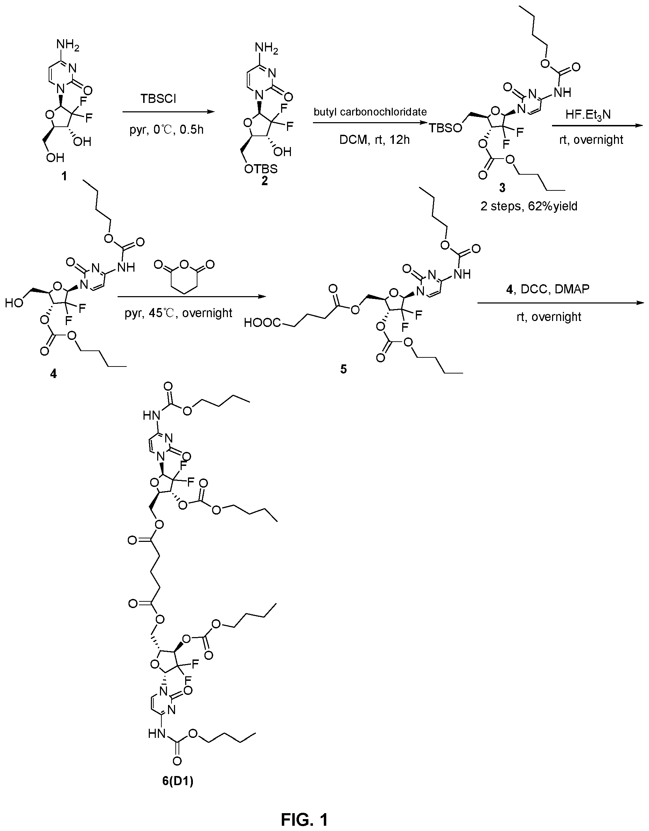

FIG. 1 illustrates a synthetic route of a cytidine derivative dimer in Example 1, in the figure TBSCI is tert-butyldimethylsilyl chloride, pyr is pyridine, DCM is dichloromethane, rt is room temperature, butyl carbonochloridate is butyl chloroformate, HF.Et₃N is triethylamine trihydrofluoride, overnight is overnight, DCC is N, N'-dicyclohexylcarbodiimide, and DMAP is 4-dimethylaminopyridine;

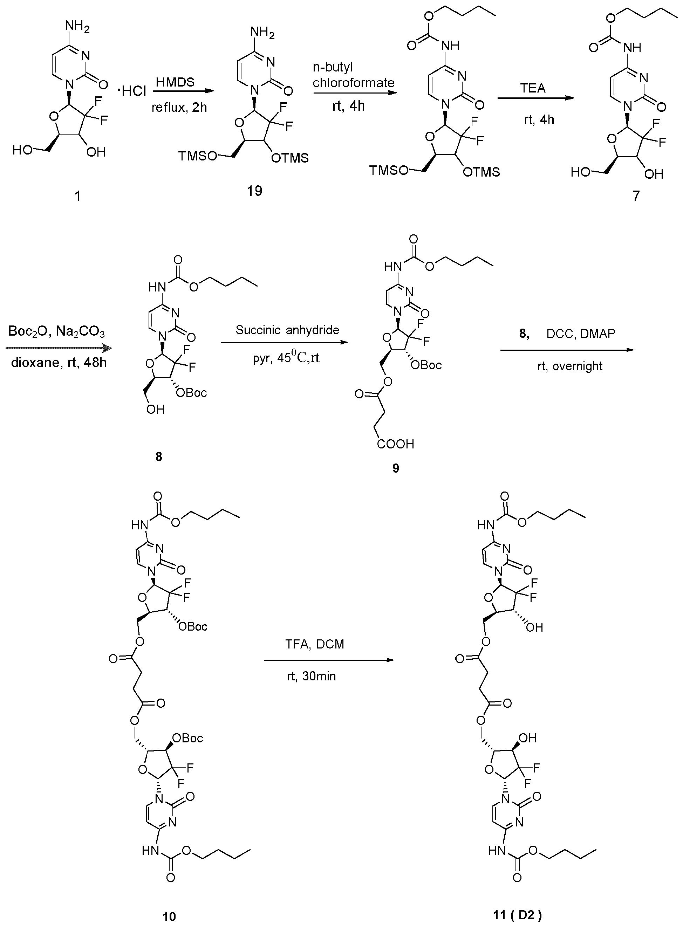

FIG. 2 illustrates a synthetic route of a cytidine derivative dimer in Example 2, in the figure HMDS is hexamethyldisilazane, reflux is reflux, chloridate is chloridate, TEA is triethylamine, (Boc)₂O is di-tert-butyl dicarbonate, dioxane is 1,4-dioxane, succinic anhydride is succinic anhydride, pyr is pyridine, DCC is N, N'-dicyclohexylcarbodiimide, DMAP is 4-dimethylaminopyridine, TFA is trifluoroacetic acid, and DCM is dichloromethane;

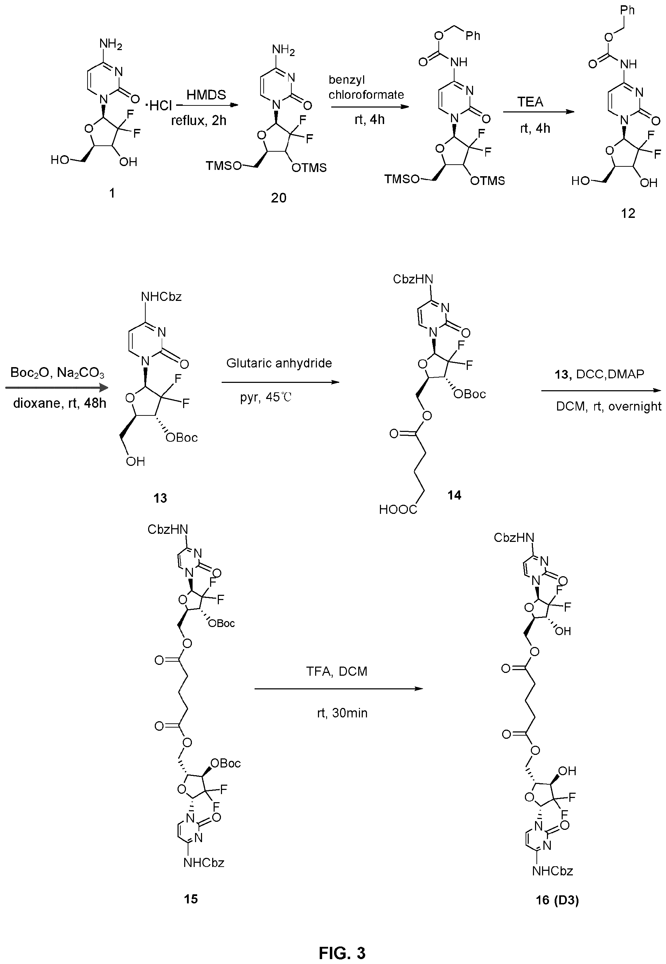

FIG. 3 illustrates a synthetic route of a cytidine derivative dimer in Example 3, in the figure HMDS is hexamethyldisilazane, reflux is reflux, chloridate is chloridate, TEA is triethylamine, (Boc)₂O is di-tert-butyl dicarbonate, dioxane is 1,4-dioxane, Glutaric anhydride is glutaric anhydride, pyr is pyridine, DCC is N, N'-dicyclohexylcarbodiimide, DMAP is 4-dimethylaminopyridine, TFA is trifluoroacetic acid, and DCM is dichloromethane;

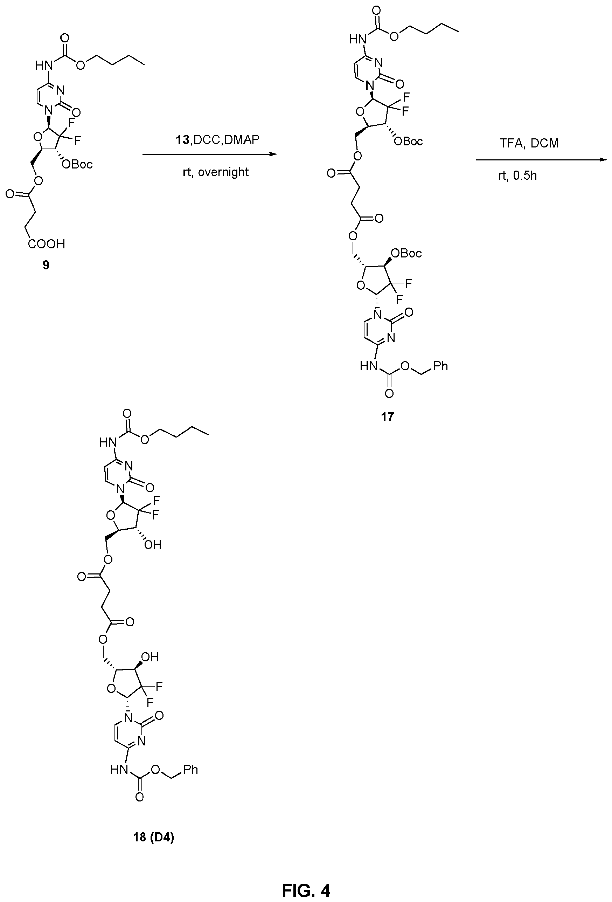

FIG. 4 illustrates a synthetic route of a cytidine derivative dimer in Example 4, in the figure DCC is **N,** N'-dicyclohexylcarbodiimide, DMAP is 4-dimethylaminopyridine, TFA is trifluoroacetic acid, and DCM is dichloromethane;

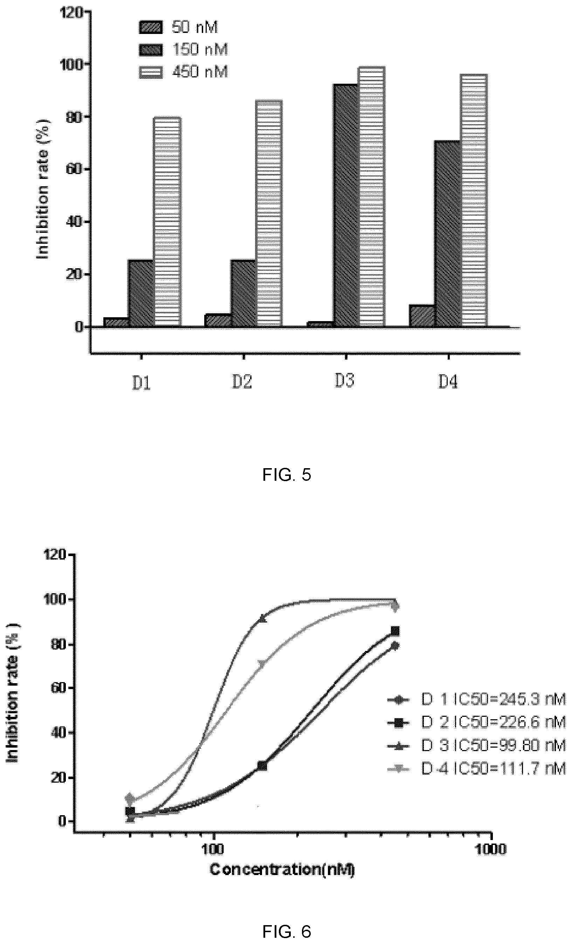

FIG. 5 is a bar chart illustrating cell clone formation inhibition rates of HCT-116 human colon cancer cells treated with four compounds at concentrations of 50 nM, 150 Nm, and 450 nM in Application Example 1; and
FIG. 6 is a curve chart illustrating a dose-response relationship between compound concentrations and inhibition rates on HCT- 116 human colon cancer cells treated with the four compounds in Application Example 1.

### DETAILED DESCRIPTION

The present invention discloses a cytidine derivative dimer with a structural formula as formula (I): 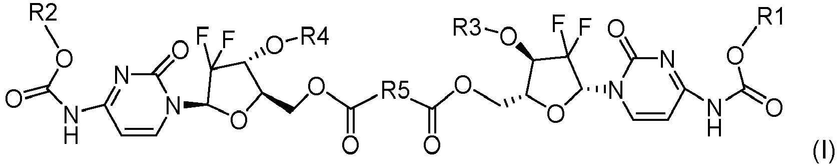 where R1 is C₁-C₁₀ alkyl, C₁-C₁₀ substituted alkyl, -(CH₂)ₙ-Ph, where n=0, 1, 2, 3∼10, or substituted -(CH₂)ₙ-Ph, where n=0, 1, 2, 3∼10, and Ph is phenyl. A carbon chain of the substituted alkyl is independently substituted by one, two, or three of halogen, cyano group, nitro group, amino group, hydroxyl group, or carboxyl group. A carbon chain or a phenyl ring of the substituted -(CH₂)ₙ-Ph is independently substituted by one, two, or three of halogen, cyano group, nitro group, amino group, hydroxyl group, or carboxyl group.

R2 is C₁-C₁₀ alkyl, C₁-C₁₀ substituted alkyl, -(CH₂)ₙ-Ph, where n=0, 1, 2, 3∼10, or substituted -(CH₂)ₙ-Ph, where n=0, 1, 2, 3∼10, and Ph is phenyl. A carbon chain of the substituted alkyl is independently substituted by one, two, or three of halogen, cyano group, nitro group, amino group, hydroxyl group, or carboxyl group. A carbon chain or a phenyl ring of the substituted -(CH₂)ₙ-Ph is independently substituted by one, two, or three of halogen, cyano group, nitro group, amino group, hydroxyl group, or carboxyl group.

R3 is hydrogen, alkoxycarbonyl, or substituted alkoxycarbonyl. A substituent of the substituted alkoxycarbonyl is halogen, cyano group, nitro group, amino group, hydroxyl group, or carboxyl group.

R4 is hydrogen, alkoxycarbonyl, or substituted alkoxycarbonyl. A substituent of the substituted alkoxycarbonyl is halogen, cyano group, nitro group, amino group, hydroxyl group, or carboxyl group.

R5 is -(CH₂)ₙ-, where n is from 1 to 15. Or R5 is substituted -(CH₂)ₙ-, the substituent of which is phenyl group, cyano group, nitro group, amino group, hydroxyl group, or carboxyl group. Alternatively, R5 is -(CH₂)ₙ-X₁-X₂-, where n=0, 1, 2, or 3, X₁ is O or S, and X₂ is -(CH₂)ₙ-Ph, where n=0, 1, 2, or 3, or X₂ is pyrimidyl, pyranyl, or pyridyl.

Regarding to cytidine derivative dimers of the present invention, Table 1 lists the following compounds.

**Table 1. Compounds of exemplary cytidine derivative dimers.**

| Compound No. | Substituent group(s) |
|---|---|
| 101 | R1 and R2 are n-butyl, R3 and R4 are n-butoxycarbonyl, R5 is-(CH₂)₃- |
| 102 | R1 and R2 are n-butyl, R3 and R4 are n-butoxycarbonyl, R5 is-CH₂- |
| 103 | R1 and R2 are n-butyl, R3 and R4 are n-butoxycarbonyl, R5 is-(CH₂)₂- |
| 104 | R1 and R2 are n-butyl, R3 and R4 are n-butoxycarbonyl, R5 is-(CH₂)₄- |
| 105 | R1 and R2 are n-butyl, R3 and R4 are n-butoxycarbonyl, R5 is-(CH₂)₅- |
| 106 | R1 and R2 are tert-butyl, R3 and R4 are n-butoxycarbonyl, R5 is-(CH₂)₃- |
| 107 | R1 and R2 are n-butyl, R3 and R4 are H, R5 is -(CH₂)₃- |
| 108 | R1 and R2 are n-butyl, R3 and R4 are H, R5 is -(CH₂)₂- |
| 109 | R1 and R2 are n-butyl, R3 and R4 are H, R5 is -(CH₂)₂-O-Ph- |
| 110 | R1 and R2 are n-butyl, R3 and R4 are H, R5 is -(CH₂)-O-Ph- |
| 111 | R1 and R2 are tert-butyl, R3 and R4 are H, R5 is -(CH₂)₂-O-Ph- |
| 112 | R1 and R2 are benzyl, R3 and R4 are H, R5 is -(CH₂)₃- |
| 113 | R1 and R2 are benzyl, R3 and R4 are H, R5 is -CHBr(CH₂)₂- |
| 114 | R1 and R2 are benzyl, R3 and R4 are H, R5 is -CHPh(CH₂)₂- |
| 115 | R1 and R2 are benzyl, R3 and R4 are H, R5 is -CHCNCH₂- |
| 116 | R1 and R2 are benzyl, R3 and R4 are H, R5 is -CHBrCH₂- |
| 117 | R1 is benzyl, R2 is n-butyl, R3 and R4 are H, R5 is -(CH₂)₂- |
| 118 | R1 is benzyl, R2 is n-butyl, R3 and R4 are H, R5 is -(CH₂)₃- |
| 119 | R1 is benzyl, R2 is n-butyl, R3 and R4 are H, R5 is -(CH₂)₂-O-Ph- |
| 120 | R1 is benzyl, R2 is n-butyl, R3 and R4 are H, R5 is -(CH₂)₂-O-Ph-, the para posistion of the phenyl ring is substituted by nitro |

When preparing the compounds in the table above, the solid reagents employed in the synthesis process are used directly without further treatment, the liquid reagents are used after redistilled and dried.

### (Example 1)

The cytidine derivative dimer of this example is 1,5-di-[4-N-(n-butyloxycarbonyl)-3'-O-(n-butoxycarbonyl)-2'-deoxy-2',2'-difluoro-cytidine]glutarate (code name D1, No. 101 in Table 1) with the following structural formula:

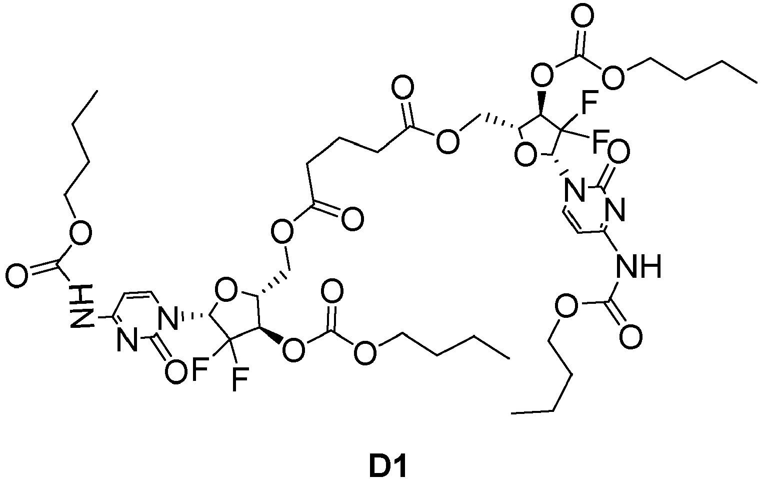

A synthetic route of D1 is shown in FIG. 1, the detailed preparation process is as follows.

2'-deoxy-2', 2'-difluoro-cytidine hydrochloride (3 g, 10 mmol) and imidazole (0.875 g, 12.8 mmol) were added into 10 mL of anhydrous pyridine, cooled in ice bath, and at 0 °C tert-butyldimethylsilyl chloride (3.3 g, 21 mmol, tert-butyldimethylsilyl chloride is abbreviated as TBSCI hereinafter) was added. The mixture was stirred for half an hour and warmed to room temperature, and the reaction was continued for 12 hours. The mixture was treated with methanol (8.0 mL). After stirring for 60 minutes, the solvent was removed under reduced pressure, and Compound 2 was obtained. The reaction system was further added with Dichloromethane (DCM) (50 mL) and pyridine (10 mL), and further added with Butyl chloroformate (5.46 g, 40 mmol) under ice bath and nitrogen protection. Such solution was stirred for 12 hours at room temperature for reaction, and then spin dried, leaving settled solid. The settled solid was dissolved in ethyl acetate (100 mL) and washed by cooled saturated sodium bicarbonate solution (30 mL for two times) and concentrated brine (30 mL). The resulting solution was dried over anhydrous sodium sulfate for 3 hours and then filtered. The filtrate was purified by column chromatography (dichloromethane/methanol 40:1) to obtain intermediate Compound 3 (3.6 g, two-step yield 62 %).

Compound 3 (3.6 g, 6.23mmol) was added into 40 mL of tetrahydrofuran (THF) and cooled in an ice bath to 0 °C. 4 mL of triethylamine trihydrofluoride (HF.Et₃N) was slowly added. After 24-hour reaction, the solvent was spin-dried in vacuum to yield an orange solid, which was purified by column chromatography (dichloromethane/methanol, 20:1) to afford intermediate Compound 4 (1.68 g, 58 % yield).

30 mL of chloroform was added with the intermediate Compound 4 (1.68 g, 3.62 mmol), added with pyridine (30 mL), and further added with glutaric anhydride (620 mg, 5.44 mmol) for an overnight reaction with stirring. Afterwards 4-dimethylaminopyridine (DMAP, 7 mg, 0.057 mmol) was further added and the mixture was stirred for 3 hours, and then concentrated to provide a viscous oily product, which was purified by column chromatography to obtain Compound 5 (1.11 g, 53 % yield).

Compound 5 (58 mg, 0.1 mmol), Compound 4 (92 mg, 0.2 mmol), and N,N'-Dicyclohexylcarbodiimide (DCC, 42 mg, 0.2 mmol) were mixed and added into dichloromethane (15 mL), DMAP (6 mg, 0.049 mmol) was further added, for a reaction at 24 °C with stirring for 24 hours. According to TLC measurement, when the reaction was measured to be completed, the mixture was added with dichloromethane (50 mL), washed by water (10 mL) and saturated brine (20 mL), dried over anhydrous sodium sulfate, and concentrated to dryness. Column chromatography (dichloromethane/methanol, 20:1) was performed to afford Compound 6 (49 mg, 48 % yield).

¹H-NMR(MeOD-d4,400MHz) δ: 7.97(d, 2H, J=7.68Hz, H6-1, H6-2), 7.40(d, 2H, J=7.68Hz, H5-1, H5-2), 6.35(t, 2H, J=7.24Hz, H1'-1, H1'-2), 4.47(m, 6H, H5a'-1, H5a'-2, H5b'-1, H5b'-2, H4'-1, H4'-2), 4.21(m, 8H, O-CH2×4), 2.53(t, 4H, J=7.16Hz, CH2-CH2-CH2), 1.97(m, 2H, CH2-CH2-CH2), 1.64(m, 8H, O-CH2-CH2×4), 1.42(m, 8H, O-CH2-CH2-CH2×4), 0.98(m, 12H, CH2-CH3×4).

¹³C NMR(MeOD-d4,100MHz) δ: 172.83, 164.51, 153.87, 144.53, 96.26, 77.52, 69.13, 65.89, 61.94, 32.42, 30.66, 30.47, 18.83, 18.67, 12.79, 12.74, 8.48.

ESIMS: calcd for C43H58F4N6O18 m/z 1023.37 (M+H)+, found 1023.66.

The aforementioned synthetic route can be adapted, by replacing glutaric anhydride with another anhydride, to produce the compounds from No. 102 to No. 105 shown in Table 1. By replacing butyl chloroformate with tert-butyl chloroformate, compound No. 106 in Table 1 can be produced.

### (Example 2)

The cytidine derivative dimer of this example is 1,5-di-[4-N-(n-butoxycarbonyl)-2'-deoxy-2',2'-difluorocytidine]glutarate (code name D2, No. 107 in Table 1) with following structural formula:

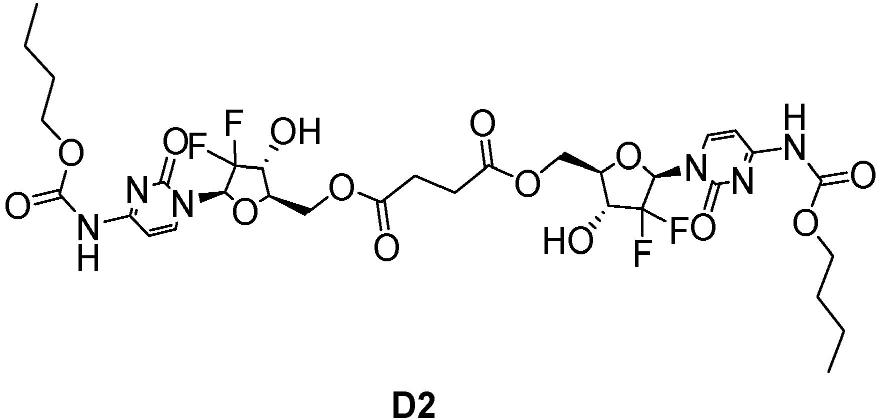

An intermediate Compound 8 was prepared first, the reaction process is shown in FIG. 2, and the preparation process is as follows.

2'- deoxy-2', 2'-difluoro-cytidine hydrochloride (Structural formula 1, 300 mg, 1 mmol), Bis(trimethylsilyl)amine HMDS (5 mL, 0.023 mmol), and 5 mg catalytic amount of ammonium sulfate were dissolved in 1,4-dioxane (5 mL). The reaction was heated under reflux for 2 hours, and the reaction product had a chemical structural formula 19. After the reaction under reflux was completed, the reaction solution was concentrated, added with toluene, and concentrated to dryness twice. The resulting product after concentration was dissolved in dichloromethane (10mL).

N-methylimidazole (0.24 mL, 3 mmol) and butyl chloroformate (0.32 mL, 3 mmol) were added into the aforementioned dichloromethane solution, for a reaction at room temperature with stirring for 4 hours. The reaction solution was then concentrated to provide a viscous oily product.

The aforementioned viscous oily product was dissolved in a mixed solution of triethylamine (3 mL) and methanol (20 mL), and stirred for 4 hours at room temperature. The solvent was removed by distillation under reduced pressure, and the crude product was purified by silica gel column chromatography with dichloromethane/methanol (20:1) to afford 230 mg of Compound 7. The three-step yield was 55.5 %.

Nuclear magnetic resonance characterization of Compound 7:
¹H-NMR (MeOD-d₄, 400MHz) δ: 8.30(d, 1H, J=7.68Hz, H6), 7.34(d, 1H, J=7.68Hz, H5), 6.28(t, 1H, J=7.08Hz, H1'), 4.33(m, 1H, H5a'), 4.0(m, 2H, O-CH₂-CH₂-), 3.81(m, 1H, H5b'), 3.79(m, 1H, H4'), 1.68(m, 2H, O-CH₂-CH₂-), 1.45(m, 2H, O-CH₂-CH₂-CH₂), 0.98(t, 3H, J=7.4Hz, -CH₂-CH₃).

¹³C-NMR(MeOD-d₄, 100MHz) δ 164.28, 156.27, 153.50, 144.39, 128.33, 122.72, 95.81, 84.90, 81.71, 74.87, 68.88, 63.69, 59.15, 30.66, 32.40, 18.81, 11.23, 8.06.

Compound 7 (60 mg, 0.16 mmol) and sodium carbonate (106 mg, 1 mmol) were mixed and added into a 5 mL solution of 1,4-dioxane and water (volume ratio 4:1). Di-tert-butyl dicarbonate (Boc)₂O (44 mg, 0.2 mmol) was added to the solution, and then stirred at 24 °C for a reaction. During the reaction, TLC was used to measure whether Compound 7 was completely reacted. After the reaction was complete, the system after reaction was diluted with water (2 mL) and extracted with ethyl acetate twice, each time with 30 mL volume. The organic phase obtained from extraction was sequentially washed by water (5 mL) and saturated saline (5 mL), dried over anhydrous sodium sulfate after the washing was completed, and then concentrated to dryness under reduced pressure. The resulting product after concentration was purified by silica gel column chromatography with dichloromethane/acetone/methanol (1:1:0.02) to afford 51 mg of Compound 8. The yield of the above reaction was 76 %.

A synthetic route of D2 is shown in FIG. 2, the preparation process is as follows.

The obtained Compound 8 (223 mg, 0.25 mmol) was dissolved in chloroform (6 mL), pyridine (5 mL) was added, and succinic anhydride (100 mg, 1 mmol) was further added. The reaction was kept overnight at 45 °C. The reaction mixture was then concentrated to viscous oil and purified by column chromatography (DCM-MeOH 20:1 to 10:1) to afford Compound 9 (211 mg, 75 % yield).

Compound 9 (56 mg, 0.1 mmol), Compound 8 (92 mg, 0.2 mmol), and DCC (42 mg, 0.2 mmol) were mixed and added into dichloromethane (15 mL), and DMAP (6 mg, 0.049 mmol) was added. The reaction was kept at 24 °C with stirring for 24 hours. According to TLC measurement, when the reaction was measured to be completed, the mixture was added with dichloromethane (50 mL), washed by water (10 mL) and saturated brine (20 mL), dried over anhydrous sodium sulfate, and concentrated to dryness. The resulting material was further added with trifluoroacetic acid (TFA, 5 mL) and dichloromethane (DCM, 10 mL) and stirred at room temperature for half an hour. After cooling with ice bath, white solid was removed by filtration. The filtrate was concentrated to obtain a viscous oil, which was purified by column chromatography (DCM-MeOH 20:1 to 10:1) to afford D2 (30 mg, 35 % yield).

¹H-NMR(MeOD-d4,400MHz) δ 7.85(d, 2H, J=7.68Hz, H6-1, H6-2), 7.37(d, 2H, J=7.68Hz, H5-1, H5-2), 6.26(t, 2H, J=7.24Hz, H1'-1, H1'-2), 4.53(m, 2H, H5a'-1, H5a'-2), 4.40(m, 4H, H5b'-1, H5b'-2, H4'-1, H4'-2), 4.20(m, 2H, H3-1, H3-2), 2.73(m, 4H, -CH2-CH2-), 1.64(m, 4H, O-CH2-CH2), 1.37(m, 4H, O-CH2-CH2-CH₂), 0.93(m, 6H, CH2-CH3).

¹³C-NMR(MeOD-d₄, 100MHz) δ 172.41, 164.01, 155.95, 153.52, 144.36, 96.56, 70.53, 66.29, 62.49, 30.74, 28.76, 19.01, 13.49.

ESIMS: calcd for C₃₂H₄₀F₄N₆O₁₄ m/z 809.25 (M+H)⁺, found 809.34.

The aforementioned preparation method can be adapted, by making Compound 8 to react with other dianhydride(s), such as glutaric anhydride, to produce the compounds from No. 108 to No. 110 shown in Table 1. By replacing butyl chloroformate with tert-butyl chloroformate, compound No. 111 shown in Table 1 can be produced.

### (Example 3)

The cytidine derivative dimer of this example is 1,5-di-[4-N-(benzyloxycarbonyl)-2'-deoxy-2',2'-difluorocytidine]glutarate (code name D3).

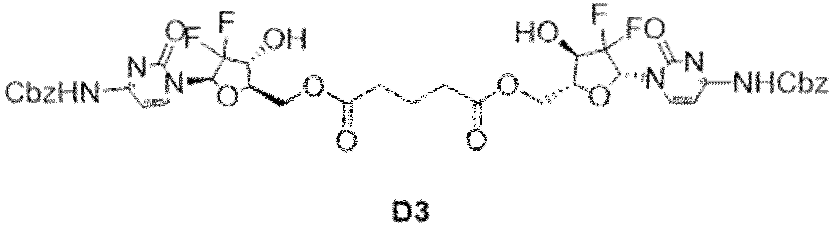

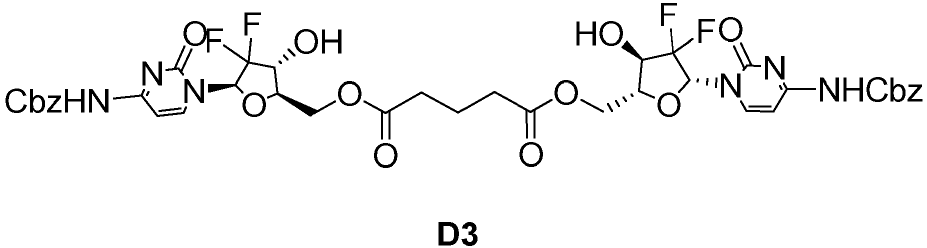

A synthetic route of D3 is shown in FIG. 3, the preparation process is as follows.

Compound 13 was prepared first: 2'-deoxy-2', 2'-difluoro-cytidine hydrochloride (300 mg, 1 mmol), Bis(trimethylsilyl)amine (5 mL, 0.023 mmol), and 5 mg catalytic amount of ammonium sulfate were dissolved in 1,4-dioxane (5 mL). The reaction was heated under reflux for 2 hours. After the reaction under reflux was completed, the reaction solution was concentrated, added with toluene, and concentrated to dryness twice. The resulting product was dissolved in dichloromethane (10 mL).

N-methylimidazole (0.24 mL, 3 mmol) and benzyl chloroformate (340 mg, 3 mmol) were added to the aforementioned dichloromethane solution, for a reaction at room temperature with stirring for 4 hours. The reaction product had a chemical structural formula 20. The reaction solution was concentrated to provide a viscous oily product.

The aforementioned viscous oily product was dissolved in a mixed solution of triethylamine (3 mL) and methanol (20 mL), and stirred at room temperature overnight. The solvent was then removed by distillation under reduced pressure. The crude product was purified by silica gel column chromatography with dichloromethane / methanol (20:1) to afford 162 mg of Compound 12. The three-step yield was 41 %.

NMR characterizations of Compound 12:
¹H-NMR (MeOD-d₄, 400MHz) δ: 8.31(d, 1H, J=7.64Hz, H6), 7.39(m, 5H, J=7.68Hz, Ph), 6.25(t, 1H, J=7.12Hz, H1'), 5.21(s, 2H. CH₂-Ph), 4.31(m, 1H, H5a'), 3.82(m, 2H, H5b, H4'), 3.79(m, 1H, H3').

¹³C-NMR (MeOD-d₄, 100MHz) δ: 164.22, 156.22, 153.27, 144.48, 135.87, 128.42, 128.10, 125.31, 122.74, 120.16, 95.89, 85.35, 84.91, 81.7, 81.66, 68.87, 67.54, 58.31.

Compound 12 (80 mg, 0.2 mmol) and sodium carbonate (106 mg, 1 mmol) were mixed and added into a system of 1,4-dioxane and water (volume ratio 4:1, 5mL). (Boc)₂O (44 mg, 0.2 mmol) was added afterwards, for a reaction at 24 °C for 48 hours with stirring. According to TLC measurement, when the reaction was measured to be completed, the reaction mixture was diluted with water (2 mL) and extracted with 30 mL ethyl acetate twice. The organic phase from extraction was washed by water (5 mL) and saturated saline (5 mL), dried over anhydrous sodium sulfate, and then concentrated to dryness under reduced pressure. The remaining residue was purified by column chromatography (dichloromethane/ acetone/ ethanol, 1:1:0.02) to afford Compound 13 (64 mg, with 64% yield).

The obtained Compound 13 (248 mg, 0.5 mmol) was added into chloroform (15 mL), pyridine (5 mL) was added, and glutaric anhydride (100 mg, 1 mmol) was further added. The reaction was kept overnight at 45 °C. The reaction mixture was then concentrated to viscous oil and purified by column chromatography (DCM-MeOH 20:1 to 10:1) to afford Compound 14 (223 mg, with 73% yield).

The obtained Compound 14 (61mg, 0.1 mmol), Compound 13 (99 mg, 0.2 mmol), and DCC (42 mg, 0.2 mmol) were mixed and added into dichloromethane (15 mL), and DMAP (6 mg, 0.049 mmol) was added. The reaction was kept at 24 °C with stirring for 24 hours. According to TLC measurement, when the reaction was measured to be completed, the mixture was added with dichloromethane (50 mL), washed by water (10 mL) and saturated brine (20 mL), dried over anhydrous sodium sulfate, and concentrated to dryness. TFA (5 mL) and DCM (10 mL) were further added, and stirred at room temperature for half an hour. After cooling with ice bath, white solid was removed by filtration. The filtrate was then concentrated to provide a viscous oil, which was purified by column chromatography (DCM-MeOH 20:1 to 10:1) to afford D3 (30 mg, with 35 % yield).

¹H-NMR (MeOD-d₄, 400MHz) δ: 8.31(d, 1H, J=7.64Hz, H6), 7.39(m, 5H, J=7.68Hz, Ph), 6.27(t, 2H, J=7.8Hz, H1'-1, H1'-2), 5.17(s, 4H, CH₂-Ph×2), 4.46(m, 4H, H5a'-1, H5a'-2, H5b'-1, H5b'-2), 4.21(m, 2H, H4'-1, H4'-2), 4.10(m, 2H, H3'-1, H3'-2), ,2.53(t, 4H, J=7.16Hz, -CH2-CH2-CH2-), 1.99(q, 2H, J=7.2Hz, -CH2-CH2-CH2-).

¹³C NMR(MeOD-d₄, 100MHz) δ 172.90, 164.21, 155.90, 153.31, 144.25, 141.03, 135.86, 128.41, 128.28, 128.10, 124.85, 123.25, 122.26, 96.14, 79.17, 74.97, 67.59, 62.06, 33.19, 32.51, 19.96.

ESIMS: calcd for C₃₉H₃₈F₄N₆O₁₄ m/z 891.24 (M+H)⁺, found 891.31.

The aforementioned preparation method can be adapted, by making Compound 14 to react with other dianhydride(s), such as COOHCHBr(CH₂)₂COOH, COOH CHPh(CH₂)₂COOH, and COOH CHCNCH₂COOH, to produce the compounds from No. 113 to No. 116 shown in Table 1.

### (Example 4)

The cytidine derivative dimer of this example is 1-O-(4-N-(Benzyloxycarbonyl)-gemcitabine)-4-O-(4-N-(n-Butoxycarbonyl)-gemcitabine)-succinate (code name D4) with the following structural formula:

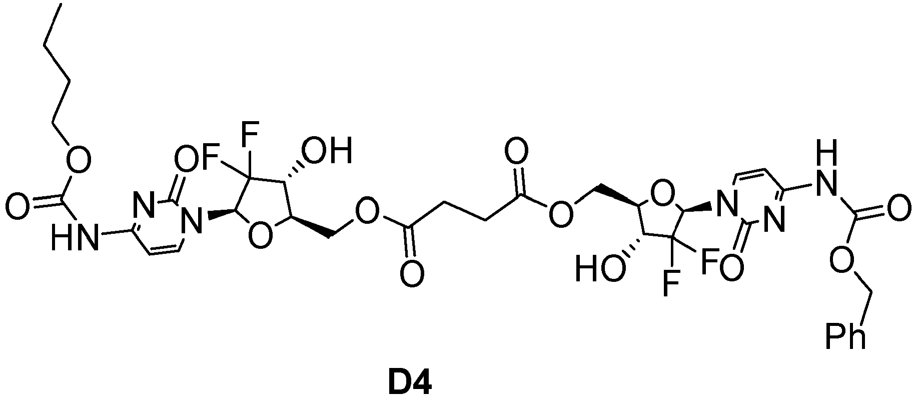

A synthetic route of D4 is shown in FIG. 4, the specific preparation process is as follows.

Compound 9 (56 mg, 0.1 mmol), Compound 13 (99 mg, 0.2 mmol), and DCC (42 mg, 0.2 mmol) were mixed and added into dichloromethane (15 mL), and DMAP (6 mg, 0.049 mmol) was added. The reaction was kept at 24 °C with stirring for 24 hours. According to TLC measurement, when the reaction was measured to be completed, the reaction mixture was added with dichloromethane (50 mL), washed by water (10 mL) and saturated brine (20 mL), dried over anhydrous sodium sulfate, and concentrated to dryness. TFA (5 mL) and DCM (10 mL) were further added, and stirred at room temperature for half an hour. After cooling with ice bath, white solid was removed by filtration. The filtrate was concentrated to provide a viscous oil and purified by column chromatography (DCM-MeOH 20:1 to 10:1) to afford D4 (30 mg, 36 % yield).

¹H-NMR(MeOD-d4,400MHz) δ 7.98(m, 2H, H6-1, H6-2), 7.40(d, 2H, J=7.68Hz, H5-1, H5-2), 7.38(m, 6H, Ph), 6.26(t, 2H, J=8Hz, H1'-1, H1'-2), 5.21 (s, 2H, CH₂-Ph), 4.43(m, 2H, H5a'-1, H5a'-2), 4.29(m, 2H, H5b'-1, H5b'-2), 4.21 (m, 6H, H4'-1, H4'-2 H3'-1, H3'-2), 2.74(m, 4H, -CH₂-CH₂-), 1.43(m, 2H, O-CH₂-CH₂-), 1.28(m, 2H, O-CH₂-CH₂-CH₂-), 0.97(t, 3H, J=7.4Hz, -CH₂-CH₃).

¹³C NMR(MeOD-d₄, 100MHz) δ 172.56, 164.19, 155.89, 153.52, 144.61, 135.86, 128.09, 122.22, 96.21, 79.38, 78.91, 70.83, 67.60, 65.92, 62.11, 56.72, 30.64, 28.66, 28.51, 25.93, 18.82, 14.26, 12.77.

ESIMS: calcd for C₃₅H₃₈F₄N₆O₁₄ m/z 843.24 (M+H)⁺, found 843.33.The aforementioned preparation method can be adapted, by replacing succinic anhydride with other anhydride(s) to react with Compound 8 and produce an intermediate compound. The intermediate compound can react with Compound 13 to produce the compounds from No. 118 to No. 120 shown in Table 1.

### (Example 5: hydrochloride of cytidine derivative dimer)

This example includes preparation of hydrochloride of the cytidine derivative dimer in Example 1.

1,5-di-[4-N-(n-butyloxycarbonyl)-3'-O-(n-butoxycarbonyl)-2'-deoxy-2',2'-difluoro-cytidine]glutarate (0.50 g) was dissolved in ethyl acetate (60 mL), placed in an ice bath and treated with dry hydrochloric acid gas. After stirring for 15 minutes, the solvent was removed to obtain a white solid product.

Similar procedure can be used to prepare hydrochloride salt of other cytidine derivative dimer(s).

In addition to the aforementioned hydrochloride salt, other salts of cytidine derivative dimer can be prepared, including phosphates, sulfates, carbonates, nitrates, citrates, tartrates, maleates, succinates, sulfonates, p-toluenesulfonates, methanesulfonates, benzoates or fumarates.

### (Example 6: Lyophilized powder of cytidine derivative dimer for injection)

This example includes preparation of injectable lyophilized powder of compound D3 in Example 3.

The injectable lyophilized powder of D3 included compound D3 (30 g), mannitol (20 %w/v, 300 g), buffer sodium dihydrogen phosphate dihydrate (7 g), and surfactant poloxamer 188 (F68) (4.0 g).

The sodium dihydrogen phosphate dihydrate, the poloxamer 188 (F68) (CAS No.: 9003-11-6), and the mannitol (20 %w/v) were weighed accurately according to the prescription amount as suggested above, and dissolved in water for injection (300 g) which was pre-cooled to a temperature lower than 10 °C. NaOH (0.1 mol/L) was used to adjust the pH value of the solution to 7.3-7.5. Prescribed dose of D3 was then added to the solution and well mixed. NaOH solution (0.1 mol/L) or HCl (0.1 mol/L) can be used to adjust the pH value to 7.3±0.2 (7.5 in this Example). The solution was added with water until 2000 g, and filtered and sterilized by 0.22 µm microporous membrane. The filtrate was dispensed in vials for 2.0 grams in each vial. The vials were partially stoppered, and placed in a freeze dryer for lyophilization. After drying, the vials were vacuum packed, capped, and labeled to prepare 1000 vials of lyophilized powder injections. The storage temperature was 2 °C - 8 °C.

In addition to the lyophilized powder injections (i.e., sterile powder for injection), the cytidine derivative dimer(s) of the present invention may be formulated in other suitable forms for injection, such as solution-type injection, suspension-type injection, emulsion-type injection.

### (Example 7: Pharmaceutical composition of cytidine derivative dimer)

An pharmaceutical composition of cytidine derivative dimer of this Example include a pharmaceutically active ingredient and an excipient. The pharmaceutically active ingredient can be the cytidine derivative dimer or corresponding salt thereof prepared in previous Examples. The weight percentage of the pharmaceutically active ingredient in the composition can be 1 % to 95 % (30 % in this Example). The excipients can include water, lactose, corn starch, hydroxypropylmethyl cellulose, and magnesium stearate. The form of the pharmaceutical composition in this Example is a tablet.

In addition to the above tablet form, the pharmaceutical composition can take other suitable forms. The pharmaceutically active ingredient can be prepared for oral administration as: powders, granules, capsules, pellets formulations, solutions, suspensions, emulsions, syrups or elixirs, or slow-controlled release formulations and controlled release formulations, or other suitable forms for oral administration. These orally administered types of pharmaceutical composition can contain commonly used corresponding excipients (categorized as additives, adjuvants, and so on depending on their functions). The additives may include mannitol, lactose, starch, magnesium stearate, sugar, salt, cellulose, magnesium sulfate, and so on in pharmaceutical grades.

When preparing the aforementioned orally administered type of pharmaceutical composition, adjuvants in pharmacology may be used as medicinal carriers for carrying the pharmaceutically active ingredients, and include materials known in the art, such as: inert solid diluents, aqueous solvents, liposomes, microspheres and/or non-toxic organic solvents. Preferred adjuvants include: wetting agents, emulsifying agents, pH buffers, human serum albumin, antioxidants, preservatives, bacteriostatic agents, dextrose, sucrose, trehalose, maltose, lecithin, glycine, sorbic acid, propylene glycol, polyethylene, protamine, boric acid, sodium chloride, potassium chloride, mineral oil, vegetable oil, etc. One or more types can be selected and combined as a medicinal carrier.

The target tumors of the disclosed pharmaceutical composition include blood tumors or malignant solid tumors. Specifically, target tumors include lung cancer, prostate cancer, breast cancer, colon cancer, stomach cancer, pancreatic cancer, liver cancer, esophageal cancer, brain tumor, ovarian cancer, uterine cancer, kidney cancer, head and neck cancer, skin cancer, bladder cancer, vulvar cancer, testicular tumor, colorectal cancer, choriocarcinoma, germ cell tumors, malignant lymphoma, leukemia and multiple myeloma. Further, preferred target tumor may include pancreatic cancer (in a first-line or second-line treatment), non-small cell lung cancer, small cell lung cancer, breast cancer, ovarian cancer and head and neck squamous cell carcinoma, and/or colon cancer.

### (Application Example 1: Inhibition test of a series of compounds on HCT-116 human colon cancer cell colony formation)

1. Colony formation inhibition test was used to evaluate the effect of four candidate compounds (D1, D2, D3, and D4) having concentrations of 50 nM, 150 nM and 450 nM on inhibiting cell proliferation of HCT-116 human colon cancer cell line.
2. Experiment material: cell lines: HCT-116 human colon cancer cell line purchased from the Chinese Academy of Sciences Shanghai Cell Resource Center, Cat # TCHu 99.
3. Reagent preparation
   HCT-116 human colon cancer cell culture medium: DMEM + 10 % FBS.
   Compound preparation: DMSO was used to dilute the compound until a final concentration of 100 µM was reached.
   Cell staining solution preparation: 0.5 % crystal violet solution was prepared using anhydrous ethanol, and stored in dark. Before staining, the solution was mixed with PBS buffer solution by volume ratio of 1:4 as the cell staining solution.
4. Cell culture: cells in logarithmic growth phase were collected, counted, and resuspended in complete culture medium. The cell concentration was adjusted to an appropriate concentration, 6-well culture plates were seeded, each well having about 300 cells and 1.8 mL of culture medium. Cells were incubated for 5 hours in an incubator at 37 °C, 100 % relative humidity, and 5 % CO₂.
5. Cell clone formation inhibition test and data processing
   (1) Cells in logarithmic growth phase were collected and counted. The cells were resuspended in culture medium containing 5 % FBS, and counted. Six-well plates were seeded by a rate of 300 cells per well. Cells were incubated for 5 hours in an incubator at 37 °C, 100% relative humidity, and 5 % CO₂.
   (2) The compounds were diluted with culture medium (containing 5% FBS) to have concentrations of 0.5 µM, 1.5 µM and 4.5 µM, and added to cells by 200 µL per well, to make final concentrations of 50 nM, 150 nM and 450 nM. Each concentration point was repeatedly tested for three times.
   (3) Cells were incubated for 72 hours in an incubator at 37 °C, 100% relative humidity, and 5 % CO₂.
   (4) The culture medium from the plates (the culture medium containing the compounds) was aspirated. The plates were rinsed with Hank's Balance Salt Solution (HBSS) twice, and replaced with fresh culture medium (DMEM medium with 15 % FBS).
   (5) Cells were incubated for 7 to 10 days in an incubator at 37 °C, 100% relative humidity, and 5 % CO₂, until formation of visible cloned plaques.
   (6) The culture medium was aspirated from the plates. The plates were rinsed twice with PBS solution.
   (7) The residual PBS was absorbed and removed, and ethanol was added at 1 mL per plate, fixing for 30 minutes.
   (8) Ethanol was absorbed and removed, and cell staining solution was added, stained for 3 minutes.
   (9) The staining solution was aspirated. After rinsing three times with PBS, the cells were counted.
   Data processing:
   Clone formation efficiency = [As / Ac] × 100 %; clone formation inhibition rate = 1-clone formation efficiency, where As denotes number of cell colonies in treated by samples (cells + compounds to be tested), and Ac denotes number of cell colonies in negative control (without treating with samples) (cells + 1 % DMSO).
6. Results and discussion

Table 2 lists numbers of cloned cells of HCT-116 human colon cancer cells treated with the four compounds.

**Table 2**

| Number of cloned HCT-116 cells | | | | | | | | |
|---|---|---|---|---|---|---|---|---|
| | DG-1 | | DG-2 | | DG-3 | | DG-4 | |
| | mean | % inhibition | mean | % inhibition | mean | % inhibition | mean | % inhibition |
| Control | 251 | | 255 | | 241 | | 256 | |
| 50nM | 242 | 3.58% | 243 | 4.70% | 237 | 1.66% | 235 | 8.20% |
| 150nM | 188 | 25.10% | 191 | 25.10% | 20 | 91.70% | 75 | 70.70% |
| 450nM | 52 | 79.28% | 36 | 85.88% | 4 | 98.34% | 11 | 95.70% |

| | | | | | | | | |
|---|---|---|---|---|---|---|---|---|
| % inhibition: inhibition rate | | | | | | | | |

FIG. 5 illustrates the cell clone formation inhibition rates of HCT-116 human colon cancer cells treated with the four compounds at concentrations of 50 nM, 150 nM and 450 nM.

Table 2 and FIG. 5 show that the disclosed compounds have significant effects in inhibiting tumor cells.

FIG. 6 illustrates dose-response relationship curves between the inhibition rates and the compound concentrations when the HCT-116 human colon cancer cells are treated with the four compounds. As shown in FIG. 6, the IC50 value of D1 was 245.3 nM, the IC50 value of D2 was 226.6 nM, the IC50 value of D3 was 99.80 nM, and the IC50 value of D4 was 111.7 nM.

### (Application Example 2: Effects of a series of compounds on tumor growth inhibition)

By observing tumor formation and growth at inoculation sites and changes in body weight of test animals, this application example evaluated tumor growth inhibition of HCT-116 colon cancer xenografts and toxicity of a single intraperitoneal injection of the compounds D1 to D4 to HCT-116 colon tumor-bearing nude mice.

1. Experiment objectives were to evaluate tumor growth inhibition of HCT-116 human colon cancer xenografts and toxicity of a single intraperitoneal injection of the disclosed cytidine derivative dimer compounds to HCT-116 colon tumor-bearing nude mice.

### 2. Preparation of the test substance

Sources of solvents used for dissolving the test substance are as follows:

| Solvent | Lot number | Supplier |
|---|---|---|
| Absolute ethanol | 10009218 | Sinopharm Chemical Reagent Co., Ltd |
| Cremophor EL | 27963 | Sigma |
| 0.9% saline | 13083004 | HUAYU Pharmaceutical Co., Ltd |

Corresponding test substance was weighed and placed into a 5 mL glass tube, and was dissolved in ethanol under the stirring of a 5 mm magnetic stirrer. After a complete dissolution, Cremophor EL was added with continuous stirring. Immediately prior to the user of the test substance, the labeled amount of physiological saline was added and stirred. The prepared ethanol, Cremophor EL, physiological saline had a volume ratio of 5:5:90.

### 3. Animals for experiments.

Varieties and strains: Balb/c nude mice; Level: specific pathogen free (SPF); Sex: female.

Supplier: Shanghai Sippr/BK Lab Animal Ltd..

Number of animals: 40 animals were ordered and ones in desired health were selected there-from for the experiments.

Certificate of animal conformity number: 0123627.

Animal age at the beginning of the experiments: 7 weeks to 9 weeks.

Animal weight at the beginning of the experiments: 18 grams to 22 grams.

Acclimatization duration: 5 days to 7 days.

Animal numbering: tail number.

The animal room was maintained at 23 ± 2 °C, 40 % - 70 % humidity, with alternating light and dark every 12 hours.

Animal foods (SLAC-M01) were purchased from Beijing Ke Ao Xie Li Cooperation Limited. Sterilized filtration water was used for the experimental animals. During the experiment period, the animals could eat and drink freely.

### 4. Experimental methods were provided.

4.1 Tumor cells: HCT-116 colon cancer cells were purchased from the Institute of Cell Biology, Chinese Academy of Sciences. F-12 medium (containing 10 % FBS) was used to culture cells at 37 °C with saturated humidity in a carbon dioxide incubator containing volume fraction of 5 % CO₂ and 95 % air. Before inoculation, cells in logarithmic growth phase were collected. After digested by 0.25 % trypsin, the cells were washed with PBS once. The cells were resuspended and counted. Serum-free medium was used for cell resuspension, and the cell concentration was adjusted to 3×10^7 cell/mL.

4.2 Animal inoculation and grouping: under aseptic conditions, each nude mice was injected with 0.1 mL of cell suspension into the right hind leg subcutaneously (3×10^6 cell/mouse). When the size of the tumor grew to have a volume of about 60-150 mm³, nude mice with a similar tumor size and a desirable shape (substantially single spherical shape, without having irregular shapes or multiple tumors grouped together) were selected and grouped, each group having mice. The grouping situation was listed as follows.

**Table 3**

| Group No. | Medication | Number of animals | Dosage (mg/kg) | Administration method | Injection |
|---|---|---|---|---|---|
| 1 | D1 | 6 | 400 | IP | QD×1 |
| 2 | D2 | 6 | 400 | IP | QD×1 |
| 3 | D3 | 6 | 350 | IP | QD×1 |
| 4 | D4 | 6 | 300 | IP | QD×1 |
| 13 | Control | 6 | - | IP | QD×1 |

| | | | | | |
|---|---|---|---|---|---|
| IP: intraperitoneal injection, QD × 1: one injection. | | | | | |

The mice in control group were injected with a mixed solution including ethanol, Cremophor EL and normal saline at a ratio of 5:5:90.

### 4.3 Drug administration and observation of animals

Tumor formation and growth at inoculation sites of nude mice in each group were observed. The diameters (D) of the tumor nodules were measured three times a week with a circle sizer ruler. In addition, the following formula was applied to calculate the volume of a tumor nodule (V): V=3/4π(D/2)³.

Evaluation indexes of antitumor activity were tumor growth inhibition rate TGI (%) and relative tumor proliferation rate T/C (%).

The calculation formula of TGI was: TGI (%) = (V_{control} - V_{Treatment}) / V_{control}) × 100 %. The relative tumor volume (RTV) was calculated by: RTV= Vt/V0, where V0 is the tumor volume at the time of group administration, and Vt is the tumor volume at the time of measurement.

The calculation formula of relative tumor proliferation rate T/C (%) was: T/C (%) = T_{RTV} / C_{RTV} × 100 %. T_{RTV} denotes treatment group RTV; and C_{RTV} denotes negative control group RTV. The mice were weighed 3 times every week.

### 4.4 Clinical symptoms

All clinical symptoms of each animal were recorded at the beginning of the experiments and during the experiments. The observation was performed at the same time every day.

After administration of the test substance, when the weight reduction was >20%, when the animal was dying, or when the tumor volume exceeds 2800 mm³, the animal was euthanized by applying CO₂. The tumor was separated and weighed, an autopsy was performed, and visual inspection was conducted and recorded on whether there were pathological changes in the organs.

### 4.5 Data and statistical analysis

Unless otherwise indicated, experimental data were presented by Mean ± SEM; unpaired T-test was used for comparing between two groups, the result was considered significantly different when P <0.05.

### 5. Experiment results

### (1) Effects of the test compounds on weight of tumor-bearing mice with human colon cancer HCT-116

**Table 4. mice weight at different days (g, Mean ± SEM)**

| Groups | Dose (mg/kg) | Day 0 (g) | Day 1 (g) | Day 2 (g) | Day 4 (g) | Day 7 (g) |
|---|---|---|---|---|---|---|
| D1 | 400 | 19.98±0.49 | 19.48±0.58 | 17.45±0.81 * | 16.15±1.08** | 19.80±1.40 |
| D2 | 400 | 19.87±0.25 | 19.85±0.28 | 18.17±0.31** | 17.75±0.44** | 18.48±0.40 |
| D3 | 350 | 19.95±0.09 | 19.23±0.31 | 17.70±0.26** | 18.24±0.58* | 19.51±0.48 |
| D4 | 300 | 20.15±0.29 | 19.90±0.19 | 18.48±0.2** | 19.07±0.36 | 19.85±0.38 |
| Control | (NA) | 20.55±0.37 | 20.43±0.45 | 20.07±0.38 | 20.02±0.29 | 19.33±0.26 |

| Groups | Dose (mg/kg) | Day 9 (g) | Day 11 (g) | Day 14 (g) | Day 16 (g) | Day 18 (g) |
|---|---|---|---|---|---|---|
| D1 | 400 | 20.75±1.05 | 21.25±1.55 | 21.35±1.35* | 21.75±1.25* | 23.30±1.60** |
| D2 | 400 | 19.92±0.45 | 20.42±0.53 | 20.85±0.60* | 21.13±0.51** | 21.63±0.51** |
| D3 | 350 | 20.74±0.38 | 21.12±0.41 | 21.26±0.21** | 22.00±0.40 | 22.58±0.38 |
| D4 | 300 | 20.73±0.29 * | 21.05±0.31 | 20.90±0.34** | 21.48±0.34 | 21.95±0.27** |
| Control | (NA) | 19.85±0.23 | 20.23±0.25 | 19.42±0.19 | 19.62±0.29 | 19.68±0.23 |

| Groups | Dose (mg/kg) | Day 21 (g) | Day 23 (g) | Day 25 (g) | Day 28 (g) | Day 30 (g) |
|---|---|---|---|---|---|---|
| D1 | 400 | 22.30±1.30** | 22.60±1.50* | 23.20±1.70 | 23.80±1.50 | 23.60±1.20 |
| D2 | 400 | 20.72±0.46* | 20.60±0.40 | 20.88±0.48 | 20.18±0.44 | 20.33±0.55 |
| D3 | 350 | 21.98±0.41 ** | 22.44±0.41** | 22.50±0.53** | 22.12±0.48* | 22.90±0.60 |
| D4 | 300 | 21.63±0.30** | 22.03±0.43** | 22.30±0.49** | 21.88±0.59 | 22.03±0.53 |
| Control | (NA) | 19.30±0.24 | 19.55±0.18 | 20.08±0.30 | 19.75±0.05 | N/A |

In the table, day means day; "*" indicates p<0.05 versus the control group; and "**" indicates p <0.01 versus the control group.

**Table 5. Weight change rates**

| Groups | Dose (mg/kg) | Day 0 (%) | Day 1 (%) | Day 2 (%) | Day 4 (%) | Day 7(%) |
|---|---|---|---|---|---|---|
| D1 | 400 | 0.00 | -2.55±0.77 | -12.18±2.63** | -18.58±4.66** | -4.23±3.77 |
| D2 | 400 | 0.00 | -0.08±0.74 | -8.54±1.31** | -10.63±2.13** | -6.98±1.43 |
| D3 | 350 | 0.00 | -3.59±0.66 | -11.25±0.96** | -8.10±2.84 | -1.68±2.50 |
| D4 | 300 | 0.00 | -1.17±1.27 | -8.18±1.56** | -5.33±1.78 | -1.43±1.92 |
| ControI | (NA) | 0.00 | -0.60±0.59 | -2.35±0.75 | -2.55±0.82 | -5.85±1.13 |

| Groups | Dose (mg/kg) | Day 9 (%) | Day 11 (%) | Day 14(%) | Day 16(%) | Day 18(%) |
|---|---|---|---|---|---|---|
| D1 | 400 | 0.42±1.92 | 2.77±4.27 | 3.29±3.29 | 5.24±2.74 | 12.70±4.20** |
| D2 | 400 | 0.21±1.42 | 2.72±1.85 | 4.87±2.08** | 6.32±1.65** | 8.83±1.43** |
| D3 | 350 | 4.50±1.93* | 6.38±1.68* | 7.12±1.19** | 10.78±0.88** | 13.71±0.63** |
| D4 | 300 | 2.91±0.55* | 4.47±0.52* | 3.72±0.73** | 6.62±0.79** | 8.99±1.38** |
| ControI | (NA) | -3.30±1.54 | -1.39±2.07 | -5.39±1.69 | -4.38±2.27 | -4.05±2.20 |

| Groups | Dose (mg/kg) | Day 21(%) | Day 23(%) | Day 25(%) | Day 28(%) | Day 30(%) |
|---|---|---|---|---|---|---|
| D1 | 400 | 7.90±2.90* | 9.32±3.82* | 12.20±4.70 | 15.14±3.64 | 14.22±2.22 |
| D2 | 400 | 4.22±1.23** | 3.66±1.05** | 5.07±1.44 | 1.57±1.50 | 2.28±1.75 |
| D3 | 350 | 10.72±1.67** | 13.08±2.31** | 13.34±2.34** | 11.44±2.29* | 15.37±2.93 |
| D4 | 300 | 7.41±1.37** | 9.37±1.82** | 10.69±2.14** | 8.62±2.68 | 9.36±2.21 |
| ControI | (NA) | -5.94±2.02 | -3.59±1.66 | -0.97±2.53 | -2.68±5.27 | N/A |

In the table, day means day; "*" indicates p<0.05 versus the control group; and "**" indicates p <0.01 versus the control group.

As indicated by data in the above tables, after intraperitoneal injecting each compound into the HCT-116 colon cancer tumor-bearing nude mice, animal weight of D1 group with a dose of 400mg/kg was significantly decreased on Day 4 of drug administration, followed by steady weight growth in later days. During Day 14 to Day 30, the weight was significantly increased comparing to the control group. There were no significant differences between other drug-treated groups and the control group.

### (2) Effects of the test compounds on tumor volume of HCT-116 human colon cancer xenografts in tumor-bearing mice

**Table 6. Detailed data about tumor volumes of each group (mm³, Mean±SEM)**

| Groups | Dose (mg/kg) | Day 0 (mm³) | Day 1 (mm³) | Day 2 (mm³) | Day 4 (mm³) |
|---|---|---|---|---|---|
| D1 | 400 | 39.36±6.16** | 58.20±17.45** | 58.20±17.45** | 37.24±9.27** |
| D2 | 400 | 53.09±9.33** | 50.78±14.16** | 55.89±18.82** | 45.96±20.25** |
| D3 | 350 | 38.77±4.33** | 36.40±3.98** | 36.40±3.98** | 23.00±3.08** |
| D4 | 300 | 44.09±5.99** | 52.03±12.91** | 52.03±12.91** | 37.37±10.46** |
| Control | (NA) | 109.55±8.60 | 180.51±9.97 | 221.87±11.44 | 296.98±22.98 |

| Groups | Dose (mg/kg) | Day 7 (mm³) | Day 9 (mm³) | Day 11 (mm³) | Day 14 (mm³) |
|---|---|---|---|---|---|
| D1 | 400 | 36.82±28.63** | 50.63±36.49** | 101.02±78.57** | 285.66±237.94* |
| D2 | 400 | 35.87±21.79** | 30.75±16.74** | 57.29±33.0** | 116.87±66.85** |
| D3 | 350 | 12.62±3.10** | 12.62±3.10** | 14.28±3.69** | 36.98±4.82** |
| D4 | 300 | 25.46±8.17** | 22.50±5.31** | 26.19±5.79** | 70.28±18.37** |
| Control | (NA) | 432.47±39.48 | 590.34±60.83 | 976.15±83.07 | 1440.15±144.73 |

| Groups | Dose (mg/kg) | Day 16 (mm³) | Day 18 (mm³) | Day 21 (mm³) | Day 23(mm³) |
|---|---|---|---|---|---|
| D1 | 400 | 495.95±408.8** | 647.07±503.28** | 1017.61±749.53 | 1017.61±749.53 |
| D2 | 400 | 169.73±88.15** | 262.10±111.14** | 489.13±174.91 | 501.98±170.25 |
| D3 | 350 | 80.10±11.07** | 120.40±23.44** | 204.05±36.25 | 229.06±36.15 |
| D4 | 300 | 142.77±49.34** | 228.58±76.92** | 375.06±86.27 | 420.73±76.08 |
| Control | (NA) | 1811.14±119. 30 | 1998.33±136.40 | 2444.84±167.64 | 2361.69±146.79 |

| Groups | Dose (mg/kg) | Day 25(mm³) | Day 28(mm³) | Day 30(mm³) | |
|---|---|---|---|---|---|
| D1 | 400 | 1044.35±722.80 | 1296.79±847.87 | 1589.29±983.15 | |
| D2 | 400 | 569.05±189.18 | 689.78±203.52 | 900.52±250.44 | |
| D3 | 350 | 266.11±39.63 | 347.17±60.84 | 479.01±75.54 | |
| D4 | 300 | 491.29±83.78 | 608.08±85.37 | 781.78±105.33 | |
| Control | (NA) | 2582.36±155.95 | 2689.30±116.86 | N/A | |

| | | | | | |
|---|---|---|---|---|---|
| "*" indicates p<0.05 versus the control group; and "**" indicates p <0.01 versus the control group. | | | | | |

As indicated by the above tumor volume data of each group, the disclosed cytidine derivative dimers significantly inhibited tumor growth.

### (3) Tumor growth inhibition rate (TGI%) of the test compounds on HCT-116 human colon cancer tumors in tumor-bearing mice

The tumor growth inhibition rate (TGI%) of the test compounds on HCT-116 human colon cancer tumors in tumor-bearing mice are shown as follows in Table 7.

**Table 7. Tumor growth inhibition rate (TGI%) of D1-D4 on HCT-116 human colon cancer tumors in tumor-bearing mice.**

| Group | Dose (mg/kg) | Day 0 (TGI%) | Day 1 (TGI%) | Day 2 (TGI%) | Day 4 (TGI%) | Day7 (TGI%) |
|---|---|---|---|---|---|---|
| D1 | 400 | 0.00 | 67.76 | 73.77 | 87.46 | 91.49 |
| D2 | 400 | 0.00 | 71.87 | 74.81 | 84.53 | 91.71 |
| D3 | 350 | 0.00 | 79.83 | 83.59 | 92.26 | 97.08 |
| D4 | 300 | 0.00 | 71.17 | 76.55 | 87.42 | 94.11 |

| Group | Dose (mg/kg) | Day 9 (TGI%) | Day 11 (TGI%) | Day 14 (TGI%) | Day 16 (TGI%) | Day 18 (TGI%) |
|---|---|---|---|---|---|---|
| D1 | 400 | 91.42 | 89.65 | 80.16 | 72.62 | 67.62 |
| D2 | 400 | 94.79 | 94.13 | 91.88 | 90.63 | 86.88 |
| D3 | 350 | 97.86 | 98.54 | 97.43 | 95.58 | 93.97 |
| D4 | 300 | 96.19 | 97.32 | 95.12 | 92.12 | 88.56 |

| Group | Dose (mg/kg) | Day 21 (TGI%) | Day 23 (TGI%) | Day 25 (TGI%) | Day 28 (TGI%) | Day 30 (TGI%) |
|---|---|---|---|---|---|---|
| D1 | 400 | 58.38 | 56.91 | 59.56 | 51.78 | 43.36 |
| D2 | 400 | 79.99 | 78.74 | 77.96 | 74.35 | 67.91 |
| D3 | 350 | 91.65 | 90.30 | 89.70 | 87.09 | 82.93 |
| D4 | 300 | 84.66 | 82.19 | 80.98 | 77.39 | 72.14 |

The tumor inhibition rate of compound D1 group with 400mg/kg dose reached a maximum value of 91.49 % on Day 7, was 72.62 % on Day 16, and was 43.36 % on Day 30. The tumor inhibition rate of compound D2 group with 400 mg/kg dose reached to a maximum value of 94.79 % on Day 9, was 90.63 % on Day 16, and was 67.91 % on Day 30. The tumor inhibition rate of compound D3 group with 350 mg/kg dose reached to a maximum value of 98.54 % on Day 11, was 95.58 % on Day 16, and was 82.93 % on Day 30. The tumor inhibition rate of compound D4 group with 300 mg/kg dose reached to a maximum value of 97.32 % on Day 11, was 92.12 % on Day 16, and was 72.14 % on Day 30.

### (4) Effects of test compounds on relative tumor volume (RTV) of HCT-116 human colon cancer tumors in tumor-bearing mice

Table 8 shows the significant effects of D1-D4 test compounds on relative tumor volume (RTV) of HCT-116 human colon cancer tumors in tumor-bearing mice, where "*" indicates p-value<0.05 versus the control group and "**" indicates p-value <0.01 versus the control group.

**Table 8. Effects of test compounds on RTV of HCT-116 human colon cancer tumors in tumor-bearing mice. (Mean ± SEM)**

| Group s | Dose (mg/kg) | Day 0 (RTV) | Day 1 (RTV) | Day2 (RTV) | Day 4 (RTV) | Day 7 (RTV) |
|---|---|---|---|---|---|---|
| D1 | 400 | 1.00 | 1.35±0.24 | 1.35±0.24* | 0.89±0.12** | 1.16±0.79* |
| D2 | 400 | 1.00 | 0.89±0.10** | 0.95±0.15** | 0.73±0.19** | 0.52±0.23** |
| D3 | 350 | 1.00 | 0.98±0.12** | 0.98±0.12** | 0.65±0.10** | 0.34±0.06** |
| D4 | 300 | 1.00 | 1.12±0.12 | 1.12±0.12 | 0.79±0.11* | 0.55±0.10* |
| Contro l | (NA) | 1.00 | 1.68±0.13 | 2.07±0.16 | 2.76±0.24 | 4.09±0.53 |

| Group s | Dose (mg/kg) | Day 9 (RTV) | Day 11 (RTV) | Day 14 (RTV) | Day 16 (RTV) | Day 18 (RTV) |
|---|---|---|---|---|---|---|
| D1 | 400 | 1.61±0.98 | 3.18±2.18 * | 8.88±6.75 | 15.44±11.56 | 20.37±13.96 |
| D2 | 400 | 0.46±0.17** | 0.87±0.35** | 1.80±0.69** | 2.66±0.88** | 4.20±1.02** |
| D3 | 350 | 0.32±0.05** | 0.37±0.09** | 1.00±0.00** | 2.17±0.14** | 3.16±0.39** |
| D4 | 300 | 0.55±0.12* | 0.69±0.20* | 1.81±0.54* | 3.29±0.82* | 5.11±1.12* |
| Contro l | (NA) | 5.61±0.83 | 9.22±1.16 | 13.84±2.21 | 17.26±2.18 | 19.09±2.45 |

| Group s | Dose (mg/kg) | Day 21 (RTV) | Day 23 (RTV) | Day25 (RTV) | Day 28 (RTV) | Day 30 (RTV) |
|---|---|---|---|---|---|---|
| D1 | 400 | 32.34±20.40 | 32.34±20.40 | 33.53±19.21 | 42±22 | 51.88±24.88 |
| D2 | 400 | 8.09±1.52 | 8.48±1.37 | 9.62±1.48 | 12.03±1.50 | 15.85±1.92 |
| D3 | 350 | 5.55±0.66 | 6.21±0.61 | 7.22±0.53 | 9.28±0.73 | 12.93±0.90 |
| D4 | 300 | 8.73±1.39 | 9.89±1.30 | 11.61±1.51 | 14.73±2.18 | 19.08±2.98 |
| Contro l | (NA) | 23.41±3.10 | 19.77±1.73 | 21.62±1.86 | 21.13±1.62 | N/A |

| | | | | | | |
|---|---|---|---|---|---|---|
| "*" indicates p<0.05 versus the control group; and "**" indicates p <0.01 versus the control group. | | | | | | |

### (5) Effects of four test compounds on relative tumor proliferation rate (T/C %) of HCT-116 human colon cancer tumors in tumor-bearing mice

Table 9 shows effects of the test compounds on relative tumor proliferation rate of HCT-116 human colon cancer tumors in tumor-bearing mice.

**Table 9. Effects of test compounds on relative tumor proliferation rate of HCT-116 human colon cancer tumors in tumor-bearing mice**

| Groups | Dose (mg/kg) | Day 0 (T/C %) | Day 1 (T/C %) | Day 2 (T/C %) | Day 4 (T/C %) | Day 7 (T/C %) |
|---|---|---|---|---|---|---|
| D1 | 400 | 0.00 | 80.13 | 65.15 | 32.20 | 28.36 |
| D2 | 400 | 0.00 | 50.15 | 40.78 | 25.19 | 16.63 |
| D3 | 350 | 0.00 | 60.98 | 49.58 | 23.26 | 6.76 |
| D4 | 300 | 0.00 | 77.07 | 66.48 | 43.31 | 31.86 |

| Groups | Dose (mg/kg) | Day 9 (T/C %) | Day 11 (T/C %) | Day 14 (T/C %) | Day 16 (T/C %) | Day 18 (T/C %) |
|---|---|---|---|---|---|---|
| D1 | 400 | 28.77 | 34.47 | 64.15 | 89.47 | 106.69 |
| D2 | 400 | 14.41 | 16.77 | 18.33 | 21.64 | 21.99 |
| D3 | 350 | 4.71 | 3.41 | 5.78 | 10.49 | 16.55 |
| D4 | 300 | 30.55 | 25.94 | 32.83 | 37.96 | 26.76 |

| Groups | Dose (mg/kg) | Day 21 (T/C %) | Day 23 (T/C %) | Day 25 (T/C %) | Day 28 (T/C %) | Day 30 (T/C %) |
|---|---|---|---|---|---|---|
| D1 | 400 | 138.14 | 163.55 | 155.09 | 198.76 | 209.10 |
| D2 | 400 | 34.57 | 42.87 | 44.51 | 56.94 | 63.87 |
| D3 | 350 | 23.70 | 31.41 | 33.40 | 43.93 | 52.09 |
| D4 | 300 | 37.28 | 50.04 | 53.68 | 69.72 | 76.91 |

The relative tumor proliferation rate of compound D1 group with 400 mg/kg dosage reached to a minimum value of 28.36 % on Day 7, and was 89.47 % on Day 16. The relative tumor proliferation rate of compound D2 group with 400 mg/kg dosage reached to a minimum value of 14.41 % on Day 9, and was 21.64 % on Day 16. The relative tumor proliferation rate of compound D3 group with 350 mg/kg dosage reached to a minimum value of 3.41 % on Day 11, and was 10.49 % on Day 16. The relative tumor proliferation rate of compound D4 group with 300 mg/kg dosage reached to a minimum value of 25.94 % on Day 11, and was 37.96 % on Day 16.

In the tumor inhibition experiment of the series of compounds on HCT-116 human colon cancer xenografts in tumor-bearing nude mice, compounds D2, D3, D4 exhibited higher tumor inhibition rates on HCT-116 human colon cancer xenografts in tumor-bearing nude mice. After a one-time intraperitoneal drug administration until Day 16, there had been a significant tumor inhibition effect without obvious influence on the body weight of the animals, indicating a high antitumor activity of the disclosed cytidine derivative dimers with very low toxic side effects.

## Claims

1. A cytidine derivative dimer, having the following general formula (I), 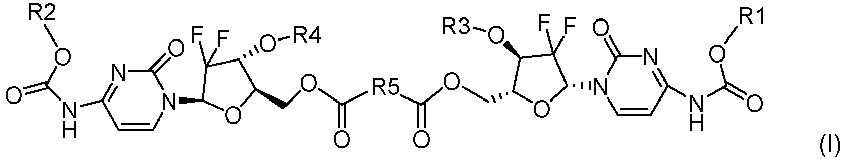 wherein:
R1 is C₁-C₁₀ alkyl, C₁-C₁₀ substituted alkyl, -(CH₂)ₙ-Ph, or substituted -(CH₂)ₙ-Ph; in the -(CH₂)ₙ-Ph, n=0, 1, 2, 3∼10, and Ph is phenyl; a carbon chain of the substituted alkyl is independently substituted by one, two, or three of halogen, cyano group, nitro group, amino group, hydroxyl group, or carboxyl group; and in the substituted -(CH₂)ₙ-Ph, n=0, 1, 2, 3∼10, and a carbon chain or a phenyl ring of which is independently substituted by one, two, or three of halogen, cyano group, nitro group, amino group, hydroxyl group, or carboxyl group;
R2 is C₁-C₁₀ alkyl, C₁-C₁₀ substituted alkyl, -(CH₂)ₙ-Ph, or substituted -(CH₂)ₙ-Ph; in the -(CH₂)ₙ-Ph, n=0, 1, 2, 3∼10, and Ph is phenyl; a carbon chain of the substituted alkyl is independently substituted by one, two, or three of halogen, cyano group, nitro group, amino group, hydroxyl group, or carboxyl group; and in the substituted -(CH₂)ₙ-Ph, n=0, 1, 2, 3∼10, and a carbon chain or a phenyl ring of which is independently substituted by one, two, or three of halogen, cyano group, nitro group, amino group, hydroxyl group, or carboxyl group;
R3 is hydrogen, alkoxycarbonyl or substituted alkoxycarbonyl, wherein a substituent of the substituted alkoxycarbonyl is halogen, cyano group, nitro group, amino group, hydroxyl group, or carboxyl group;
R4 is hydrogen, alkoxycarbonyl or substituted alkoxycarbonyl, wherein a substituent of the substituted alkoxycarbonyl is halogen, cyano group, nitro group, amino group, hydroxyl group, or carboxyl group; and
R5 is -(CH₂)ₙ-, n being from 1 to 15, or substituted -(CH₂)ₙ- with a substituent on a carbon chain thereof, the substituent is phenyl group, substituted phenyl group, cyano group, nitro group, amino group, hydroxyl group, carboxyl group, or -(CH₂)ₙ-X₁-X₂-; in the -(CH₂)ₙ-X₁-X₂-, n=0, 1, 2, or 3, X₁ is O or S, and X₂ is -(CH₂)ₙ-Ph, n being 0, 1, 2, or 3, or X₂ is pyrimidyl, pyranyl, imidazolyl, pyrazinyl, or pyridyl.

2. The cytidine derivative dimer according to claim 1, **characterized in that**: R3 is hydrogen and R4 is hydrogen.

3. The cytidine derivative dimer according to claim 2, **characterized in that**: R1 and R2 are same.

4. The cytidine derivative dimer according to claim 1 or a salt form thereof for use in a method of tumor inhibition.

5. The cytidine derivative dimer or a salt form thereof for use according to claim 4, wherein the tumor is a blood tumor or a malignant solid tumor.

6. A pharmaceutical composition, comprising the cytidine derivative dimer as shown by the general formula (I) according to claim 1 or a pharmaceutically acceptable salt thereof as an active ingredient; and one or more of medicinal carriers or excipients.

7. The pharmaceutical composition according to claim 6, **characterized in that**: a form of the pharmaceutical composition is an injection form, or an oral administration form, wherein:
the oral administration form includes tablet, powder, granule, capsule, pellet formulation, solution, suspension, emulsion, syrup, or elixir; and
the injection form includes a solution-type injection, a suspension-type injection, an emulsion injection, or a sterile powder-type injection.

8. A method for preparing the cytidine derivative dimer according to claim 1, **characterized in that** the method comprises the following steps:
1) preparing a compound having a general formula (II); 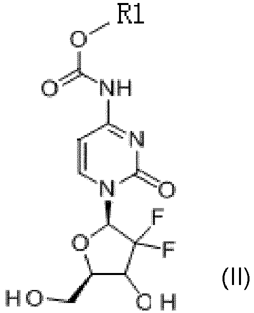
2) preparing a compound having a general formula (III); 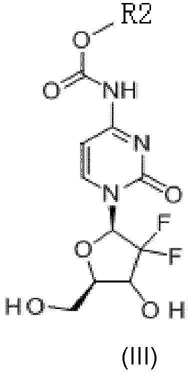
3) mixing the compound having the general formula (II) with sodium carbonate to add to a system including 1,4-dioxane and water, then adding (Boc)₂O for a reaction, and according to TLC measurement, when the reaction is measured to be completed to provide a reaction product, the reaction product being extracted, washed, and dried, and then concentrated to dryness under a reduced pressure; and adding the dried compound into chloroform, adding in pyridine and dianhydrides R₅(CO)₂O for an overnight reaction, concentrating to yield a viscous oil, purifying the viscous oil by column chromatography to obtain a compound with a general formula (IV) 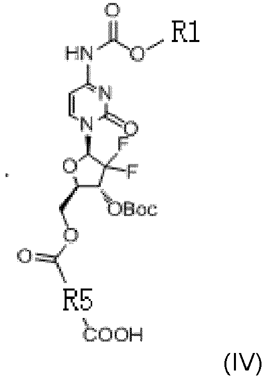
(4) after mixing the compound having the general formula (IV), the compound having the general formula (III), and DCC, adding the mixture to dichloromethane, and adding in DMAP for a reaction; according to TLC measurement, when the reaction is measured to be completed to provide a reaction product, the reaction product being washed, dried, and concentrated to dryness; further adding TFA and DCM, stirring at room temperature; after cooling with ice bath, filtering out a white solid, and concentrating the filtrate to obtain a viscous oil, which is purified by column chromatography to produce the cytidine derivative dimer.

## Patentansprüche

1. Cytidinderivat-Dimer mit der folgenden allgemeinen Formel (I) 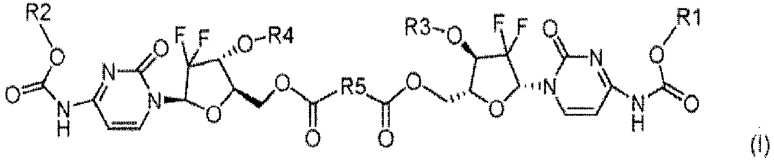 worin:
R1 für C₁-C₁₀-Alkyl, substituiertes C₁-C₁₀-Alkyl, -(CH₂)ₙ-Ph oder substituiertes - (CH₂)ₙ-Ph steht; in dem -(CH₂)ₙ-Ph, n = 0, 1, 2, 3∼10 und Ph Phenyl ist; eine Kohlenstoffkette des substituierten Alkyls unabhängig durch ein, zwei oder drei aus Halogen, Cyanogruppe, Nitrogruppe, Aminogruppe, Hydroxygruppe oder Carboxygruppe substituiert ist; und in dem substituierten -(CH₂)ₙ-Ph, n = 0, 1, 2, 3∼10 ist und eine Kohlenstoffkette oder ein Phenylring davon unabhängig durch ein, zwei oder drei aus Halogen, Cyanogruppe, Nitrogruppe, Aminogruppe, Hydroxygruppe oder Carboxygruppe substituiert ist;
R2 für C₁-C₁₀-Alkyl, substituiertes C₁-C₁₀-Alkyl, -(CH₂)ₙ-Ph oder substituiertes - (CH₂)ₙ-Ph steht; in dem -(CH₂)ₙ-Ph, n = 0, 1, 2, 3∼10 und Ph Phenyl ist; eine Kohlenstoffkette des substituierten Alkyls unabhängig durch ein, zwei oder drei aus Halogen, Cyanogruppe, Nitrogruppe, Aminogruppe, Hydroxygruppe oder Carboxygruppe substituiert ist; und in dem substituierten -(CH₂)ₙ-Ph, n = 0, 1, 2, 3∼10 ist und eine Kohlenstoffkette oder ein Phenylring davon unabhängig durch ein, zwei oder drei aus Halogen, Cyanogruppe, Nitrogruppe, Aminogruppe, Hydroxygruppe oder Carboxygruppe substituiert ist;
R3 Wasserstoff, Alkoxycarbonyl oder substituiertes Alkoxycarbonyl ist, wobei ein Substituent des substituierten Alkoxycarbonyls Halogen, Cyanogruppe, Nitrogruppe, Aminogruppe, Hydroxygruppe oder Carboxygruppe ist;
R4 Wasserstoff, Alkoxycarbonyl oder substituiertes Alkoxycarbonyl ist, wobei ein Substituent des substituierten Alkoxycarbonyls Halogen, Cyanogruppe, Nitrogruppe, Aminogruppe, Hydroxygruppe oder Carboxygruppe ist; und
R5 für -(CH₂)ₙ-, wobei n von 1 bis 15 ist, oder substituiertes -(CH₂)ₙ- mit einem Substituenten an einer Kohlenstoffkette davon steht, wobei der Substituent eine Phenylgruppe, substituierte Phenylgruppe, Cyanogruppe, Nitrogruppe, Aminogruppe, Hydroxygruppe, Carboxygruppe oder -(CH₂)ₙ-X₁-X₂ ist; in dem -(CH₂)ₙ-X₁-X₂, n = 0, 1, 2 oder 3 ist, X₁ für O oder S steht, und X₂ für -(CH₂)ₙ-Ph steht, wobei n 0, 1, 2 oder 3 ist, oder X₂ Pyrimidyl, Pyranyl, Imidazolyl, Pyrazinyl oder Pyridyl ist.

2. Das Cytidinderivat-Dimer gemäß Anspruch 1, **dadurch gekennzeichnet dass**: R3 Wasserstoff ist und R4 Wasserstoff ist.

3. Das Cytidinderivat-Dimer gemäß Anspruch 2, **dadurch gekennzeichnet dass**: R1 und R2 gleich sind.

4. Das Cytidinderivat-Dimer gemäß Anspruch 1 oder ein Salz davon, zur Verwendung in einem Verfahren zur Tumorhemmung.

5. Das Cytidinderivat-Dimer oder ein Salz davon zur Verwendung gemäß Anpsruch 4, wobei der Tumor ein Bluttumor oder ein maligner fester Tumor ist.

6. Pharmazeutische Zusammensetzung, umfassend das Cytidinderivat-Dimer, wie in der allgemeinen Formel (I) gemäß Anspruch 1 gezeigt, oder ein pharmazeutisch akzeptables Salz davon, als einen Wirkstoff; und einen oder mehrere medizinische Träger oder Hilfsstoffe.

7. Die pharmazeutische Zusammensetzung gemäß Anspruch 6, **dadurch gekennzeichnet dass**: eine Form der pharmazeutischen Zusammensetzung eine Injektionsform oder eine orale Verabreichungsform ist, wobei:
die orale Verabreichungsform ein/eine Tablette, Pulver, Granulat, Pelletformulierung, Lösung, Suspension, Emulsion, Sirup oder Elixier umfasst; und
die Injektionsform eine lösungsartige Injektion, eine suspensionsartige Injektion, eine Emulsionsinjektion oder eine sterile pulverartige Injektion umfasst.

8. Verfahren zur Herstellung des Cytidinderivat-Dimers gemäß Anspruch 1, **dadurch gekennzeichnet dass** das Verfahren die folgenden Schritte umfasst:
1) Herstellen einer Verbindung mit der allgemeinen Formel (II): 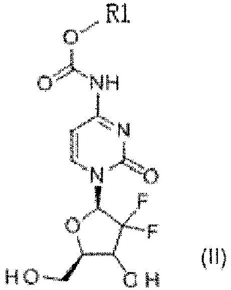
2) Herstellen einer Verbindung mit der allgemeinen Formel (III): 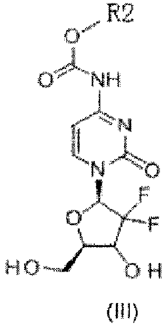
3) Mischen der Verbindung mit der allgemeinen Formel (II) mit Natriumcarbonat zur Zugabe zu einem System, das 1,4-Dioxan und Wasser umfasst, dann Zugeben von (Boc)₂O für eine Reaktion, und wenn, gemäß einer TLC-Messung, die Reaktion als abgeschlossen gemessen wird, zur Bereitstellung eines Reaktionsprodukts, wobei das Reaktionsprodukt extrahiert, gewaschen und getrocknet, und dann bis zur Trockne unter einem reduzierten Druck konzentriert wird; und Hinzufügen der getrockneten Verbindung zu Chloroform, Einbringen in Pyridin und Dianhydride R₅(CO)₂O für eine Übernacht-Reaktion, Konzentrieren zum Erhalt eines viskösen Öls, Reinigen des viskösen Öls durch Säulenchromatographie zum Erhalt einer Verbindumg mit der allgemeinen Formel (IV) 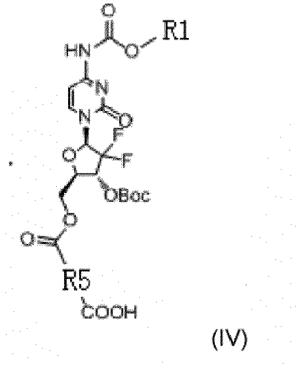
4) nach Vermischen der Verbindung mit der allgemeinen Formel (IV), der Verbindung mit der allgemeinen Formel (III) und DCC, Hinzufügen des Gemischs zu Dichlormethan, und Einbringen in DMAP für eine Reaktion; wenn, gemäß einer TLC-Messung, die Reaktion als abgeschlossen gemessen wird, zur Bereitstellung eines Reaktionsprodukts, wobei das Reaktionsprodukt gewaschen, getrocknet und bis zur Trockne konzentriert wird; weiter Hinzufügen von TFA und DCM, Rühren bei Raumtemperatur; nach Abkühlen mit einem Eisbad, Herausfiltern eines weißen Feststoffs und Konzentrieren des Filtrats zum Erhalt eines viskösen Öls, das durch Säulenchromatographie gereinigt wird, zum Erhalt des Cytidinderivat-Dimers.

## Revendications

1. Dimère dérivé de cytidine, ayant la formule générale (I) suivante : 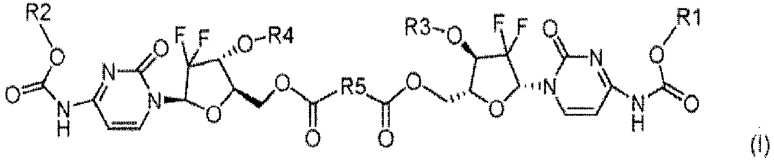 dans laquelle :
R1 est un groupe alkyle en C₁ à C₁₀, alkyle en C₁ à C₁₀ substitué, -(CH₂)ₙ-Ph ou - (CH₂)ₙ-Ph substitué ; dans le groupe -(CH₂)ₙ-Ph, n = 0, 1, 2, 3 ∼ 10 et Ph est un groupe phényle ; une chaîne carbonée du groupe alkyle substitué est indépendamment substituée par un, deux ou trois d'un atome d'halogène, d'un groupe cyano, d'un groupe nitro, d'un groupe amino, d'un groupe hydroxyle ou d'un groupe carboxyle ; et dans le groupe -(CH₂)ₙ-Ph substitué, n = 0, 1, 2, 3 ∼ 10 et une chaîne carbonée ou un cycle phényle qui est indépendamment substitué par un, deux ou trois d'un atome d'halogène, d'un groupe cyano, d'un groupe nitro, d'un groupe amino, d'un groupe hydroxyle ou d'un groupe carboxyle ;
R2 est un groupe alkyle en C₁ à C₁₀, alkyle en C₁ à C₁₀ substitué, -(CH₂)ₙ-Ph, - (CH₂)ₙ-Ph substitué ; dans le groupe -(CH₂)ₙ-Ph, n = 0, 1, 2, 3 ∼ 10 et Ph est un groupe phényle ; une chaîne carbonée du groupe alkyle substitué est indépendamment substituée par un, deux ou trois d'un atome d'halogène, d'un groupe cyano, d'un groupe nitro, d'un groupe amino, d'un groupe hydroxyle ou d'un groupe carboxyle ; et dans le groupe -(CH₂)ₙ-Ph substitué, n = 0, 1, 2, 3 ∼ 10 et une chaîne carbonée ou un cycle phényle qui est indépendamment substitué par un, deux ou trois d'un atome d'halogène, d'un groupe cyano, d'un groupe nitro, d'un groupe amino, d'un groupe hydroxyle ou d'un groupe carboxyle ;
R3 est un atome d'hydrogène, un groupe alcoxycarbonyle ou alcoxycarbonyle substitué, un substituant du groupe alcoxycarbonyle substitué étant un atome d'halogène, un groupe cyano, un groupe nitro, un groupe amino, un groupe hydroxyle ou un groupe carboxyle ;
R4 est un atome d'hydrogène, un groupe alcoxycarbonyle ou alcoxycarbonyle substitué, un substituant du groupe alcoxycarbonyle substitué étant un atome d'halogène, un groupe cyano, un groupe nitro, un groupe amino, un groupe hydroxyle ou un groupe carboxyle ; et
R5 est un groupe -(CH₂)ₙ-, n valant de 1 à 15, ou un groupe -(CH₂)ₙ- substitué avec un substituant sur une de ses chaîne carbonées, le substituant est un groupe phényle, un groupe phényle substitué, un groupe cyano, un groupe nitro, un groupe amino, un groupe hydroxyle, un groupe carboxyle ou un groupe -(CH₂)ₙ-X₁-X₂- ; dans le groupe -(CH₂)ₙ-X₁-X₂-, n = 0, 1, 2 ou 3, X1 est O ou S, et X₂ est -(CH₂)ₙ-Ph, n valant 0, 1, 2 ou 3 ou X₂ est un groupe pyrimidyle, pyranyle, imidazolyle, pyrazinyle ou pyridyle.

2. Dimère de dérivé de cytidine selon la revendication 1, **caractérisé en ce que** : R3 est un atome d'hydrogène et R4 est un atome d'hydrogène.

3. Dimère dérivé de cytidine selon la revendication 2, **caractérisé en ce que** : R1 et R2 sont identiques.

4. Dimère de dérivé de cytidine selon la revendication 1 ou une de ses formes salines destiné à être utilisé dans un procédé d'inhibition de tumeur.

5. Dimère de dérivé de cytidine ou une de ses formes salines destiné à être utilisé selon la revendication 4, dans lequel la tumeur est une tumeur sanguine ou une tumeur solide maligne.

6. Composition pharmaceutique, comprenant le dimère de dérivé de cytidine tel que montré par la formule générale (I) selon la revendication 1 ou un de ses sels pharmaceutiquement acceptables comme ingrédient actif ; et un ou plusieurs véhicules ou excipients médicinaux.

7. Composition pharmaceutique selon la revendication 6, **caractérisée en ce que** : une forme de la composition pharmaceutique est une forme pour injection, ou une forme d'administration orale :
la forme d'administration orale comprenant un comprimé, une poudre, un granulé, une capsule, une formulation de comprimé, une solution, une suspension, une émulsion, un sirop ou un élixir ; et
la forme pour injection comprenant une injection de type solution, une injection de type suspension, une injection d'émulsion ou une injection de type poudre stérile.

8. Procédé de préparation de dimère de dérivé de cytidine selon la revendication 1, **caractérisé en ce que** le procédé comprend les étapes suivantes consistant à :
1) préparer un composé ayant une formule générale (II) : 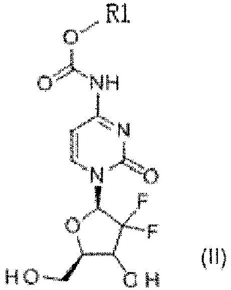
2) préparer un composé ayant une formule générale (III) ; 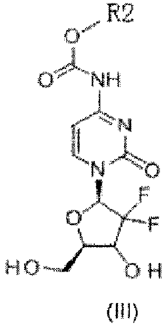
3) mélanger le composé ayant la formule générale (II) avec du carbonate de sodium à ajouter à un système comprenant du 1,4-dioxanne et de l'eau, puis ajouter (Boc)₂O pour une réaction, et selon une mesure de CCM, quand la réaction est mesurée pour être complétée pour fournir un produit réactionnel, le produit réactionnel étant extrait, lavé et séché, puis concentré à l'état sec sous pression réduite ; et ajouter le composé séché dans du chloroforme, ajouter dans de la pyridine et des dianhydrides R5(CO)₂O pour une réaction pendant la nuit, concentrer pour donner une huile visqueuse, purifier l'huile visqueuse par chromatographie sur colonne pour obtenir un composé avec une formule générale (IV) 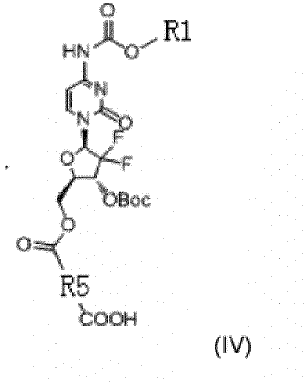
4) après le mélange du composé ayant la formule générale (IV), le composé ayant la formule générale (III), et du DCC, ajouter le mélange à du dichlorométhane, et ajouter dans du DMAP pour une réaction ; selon la mesure de CCM, quand la réaction est mesurée pour être complétée pour fournir un produit réactionnel, le produit réactionnel étant lavé, séché et concentré à l'état sec ; ajouter en outre du TFA et du DCM, agiter à température ambiante ; après refroidissement avec un bain de glace, filtrer un solide blanc et concentrer le filtrat pour obtenir une huile visqueuse, qui est purifiée par chromatographie sur colonne pour produire le dimère de dérivé de cytidine.
